(19) Europäisches Patentamt
European Patent Office
Office européen des brevets

(11) **EP 4 213 705 B1**

(12) **EUROPEAN PATENT SPECIFICATION**

(45) Date of publication and mention
of the grant of the patent:
**06.11.2024   Bulletin 2024/45**

(21) Application number: **21773845.9**

(22) Date of filing: **17.09.2021**

(51) International Patent Classification (IPC):
**A61B 5/00** *(2006.01)*       **A61B 5/026** *(2006.01)*

(52) Cooperative Patent Classification (CPC):
**G02B 27/48; A61B 5/0261; A61B 5/489;**
**A61B 5/7207; A61B 5/725; G02B 21/0012;**
A61B 2576/00

(86) International application number:
**PCT/EP2021/075620**

(87) International publication number:
**WO 2022/058499 (24.03.2022 Gazette 2022/12)**

(54) **MOTION-COMPENSATED LASER SPECKLE CONTRAST IMAGING**

BEWEGUNGSKOMPENSIERTE LASER-SPECKLE-KONTRAST-BILDGEBUNG

IMAGERIE DE CONTRASTE PAR SPECKLE LASER À COMPENSATION DE MOUVEMENT

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority:   **18.09.2020   NL 2026505**

(43) Date of publication of application:
**26.07.2023   Bulletin 2023/30**

(73) Proprietor: **LIMIS Development B.V.**
**8934 AD Leeuwarden (NL)**

(72) Inventors:
  • **DIJKSTRA, Klaas**
    **8900 CB Leeuwarden (NL)**
  • **CALON, Joost, Emiel, Marcel**
    **8401 DK Gorredijk (NL)**
  • **VERMEULEN, Arend, Jan, Wilhelmus, Abraham**
    **9222 NZ Drachtster Compagnie (NL)**
  • **BOERMA, Evert, Christiaan**
    **8934 AD Leeuwarden (NL)**

(74) Representative: **De Vries & Metman**
**Overschiestraat 180**
**1062 XK Amsterdam (NL)**

(56) References cited:
**EP-A1- 1 324 051        EP-A2- 2 473 102**
**US-A1- 2017 181 636    US-A1- 2019 046 232**
**US-A1- 2021 219 961**

  • **PENG MIAO ET AL: "High Resolution Cerebral**
    **Blood Flow Imaging by Registered Laser Speckle**
    **Contrast Analysis", IEEE TRANSACTIONS ON**
    **BIOMEDICAL ENGINEERING, IEEE, USA, vol. 57,**
    **no. 5, 1 May 2010 (2010-05-01), pages 1152 - 1157,**
    **XP011343226, ISSN: 0018-9294, DOI:**
    **10.1109/TBME.2009.2037434**

**Description**

Field of the invention

The disclosure relates to motion-compensated laser speckle contrast imaging, and, in particular, though not exclusively, to methods and systems for motion-compensated laser speckle contrast imaging, a module for motion-compensation in a laser speckle contrast imaging systems and methods and a computer program product enabling a computer system to perform such methods.

Background of the invention

Laser speckle contrast imaging (LSCI) provides a fast, full-field, and *in vivo* imaging method for determining two-dimensional (2D) perfusion maps of living biological tissue. Perfusion can be an indicator for tissue viability and thus may provide valuable information for diagnostics and surgery. For example, during a bowel operation, selection of a high-perfused intervention site may reduce anastomotic leakage.

LSCI is based on the principle that the backscattered light from a tissue illuminated with coherent laser light forms a random interference pattern at the detector due to differences in optical path lengths. The resulting interference pattern is called a speckle pattern, and may be imaged in real-time using a digital camera. Movement of particles inside the tissue causes fluctuations in this speckle pattern resulting in blurring of the speckles in those parts of the images where perfusion takes place.

For example, this blurring may be related to blood flow if the fluctuations are caused by the movement of red blood cells. This way, blood perfusion can be imaged in living tissue in a relatively simple way. Examples of state of the art clinical perfusion imaging schemes by LSCI are described in the review article by W. Heeman et al, 'Clinical applications of laser speckle contrast imaging: a review', J. Biomed. Opt. 24:8 (2019). Perfusion by other bodily fluids, e.g. lymph perfusion, may be imaged in a similar way.

LSCI, however, is extremely sensitive to any type of motion. Blurring may not only be caused by movement of blood flow but also any other type of motion such as movement of tissue due to respiration, heartbeat, muscle contraction or to motion of the camera, especially in handheld cameras.

In many medical applications, such as diagnostics and surgery, it is desired that the LSCI system is capable of generating accurate, high-resolution blood flow images, in particular microcirculation images, in real-time, in which motion artefacts are substantially reduced. Hence, during the processing of the raw speckle images, measures are required to minimize motion artefacts so that accurate, high resolution perfusion images can be acquired. This may improve identification of well-perfused and poorly perfused areas, and thus increase diagnosis and treatment outcome.

Various schemes for reducing motion artefacts in speckle images are known in the prior art. For example, WO2020/045015 A1 discloses a laser speckle contrast imaging system which is capable of capturing near-infrared speckle images and white light images of an imaging target. A simple motion detection scheme may include the use of a reference marker on an image target, tracking a feature point in the visible light images, or a change in a speckle shape in a speckle image to determine a global motion vector indicating an amount of movement of an image target between two subsequent images. Speckle contrast images may be generated based on the speckle images and can be corrected for the amount of motion based on the motion vector.

Motion will even be more prominent in handheld LSCI systems, compared to e.g. tripod-supported systems. For example, Lertsakdadet et al, described in their article 'Correcting for motion artefact in handheld laser speckle images', Journal of biomedical optics 23(2), March 2018, a motion compensation scheme for laser speckle imaging using a fiducial marker that is attached to the tissue that needs to be imaged. The use of a marker is not possible in many applications.

Similarly, P. Miao et al, describe in their article 'High resolution cerebral blood flow imaging by registered laser speckle contrast analysis', IEEE transactions on bio-medical engineering 57(5): 1152-1157, a method of producing high-resolution LSCI images by registering raw speckle images based on convolutional filtering and a correlation and interpolation scheme. The registered images are subsequently analysed retrospectively using temporal laser speckle contrast analysis. Such registration of raw speckle images however requires large computational resources and thus are not suitable for accurate real-time imaging applications.

US 2019/046232 A1 describes a method for marker-based image registration for medical images.

EP 1324051 A1 describes an endoscopic apparatus for laser speckle contrast imaging.

Hence, from the above it follows that there is a need in the art for improved motion-compensated laser speckle contrast imaging schemes. In particular, there is a need in the art for improved methods and systems for laser speckle contrast imaging that allows realization of real-time, robust, markerless, high-resolution perfusion imaging, in particular microcirculation imaging, in which motion artefacts are substantially eliminated, or at least reduced.

## Summary of the invention

As will be appreciated by one skilled in the art, aspects of the present invention may be embodied as a system, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system". Functions described in this disclosure may be implemented as an algorithm executed by a microprocessor of a computer. Furthermore, aspects of the present invention may take the form of a computer program product embodied in one or more computer readable medium(s) having computer readable program code embodied, e.g., stored, thereon.

Any combination of one or more computer readable medium(s) may be utilized. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. More specific examples (a non-exhaustive list) of the computer readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer readable storage medium may be any tangible medium that can contain, or store a program for use by or in connection with an instruction execution system, apparatus, or device.

A computer readable signal medium may include a propagated data signal with computer readable program code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

Program code embodied on a computer readable medium may be transmitted using any appropriate medium, including but not limited to wireless, wireline, optical fiber, cable, RF, etc., or any suitable combination of the foregoing. Computer program code for carrying out operations for aspects of the present invention may be written in any combination of one or more programming languages, including a functional or an object oriented programming language such as Java(TM), Scala, C++, Python or the like and conventional procedural programming languages, such as the "C" programming language or similar programming languages. The program code may execute entirely on the user's computer, partly on the user's computer, as a stand-alone software package, partly on the user's computer and partly on a remote computer, or entirely on the remote computer, server or virtualized server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described below with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer program instructions. These computer program instructions may be provided to a processor, in particular a microprocessor or central processing unit (CPU), or graphics processing unit (GPU), of a general purpose computer, special purpose computer, or other programmable data processing apparatus to produce a machine, such that the instructions, which execute via the processor of the computer, other programmable data processing apparatus, or other devices create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

The flowchart and block diagrams in the figures illustrate the architecture, functionality, and operation of possible implementations of systems, methods and computer program products according to various embodiments of the present invention. In this regard, each block in the flowchart or block diagrams may represent a module, segment, or portion of code, which comprises one or more executable instructions for implementing the specified logical function(s). It should also be noted that, in some alternative implementations, the functions noted in the blocks may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustrations, and combinations of blocks in the block diagrams and/or flowchart illustrations, can be implemented by special purpose hardware-based systems that perform the specified functions or acts, or combinations of special purpose hardware and computer instructions.

It is an objective of the embodiments in this disclosure to reduce or eliminate at least one of the drawbacks known in the prior art.

In a first aspect, the invention relates to a method of motion-compensated laser speckle contrast imaging. The method comprises exposing a target area to coherent first light of a first wavelength, the target area including living tissue, and capturing at least one sequence of images. The at least one sequence of images comprises first speckle images, the first speckle images being captured during the exposure with the first light. The method further comprises determining one or more transformation parameters of an image registration algorithm for registering the first speckle images with each other. The transformation parameters may be based on a similarity measure of pixel values of at least one group of pixels in each of a plurality of images of the at least one sequence of images, the images in the plurality of images being selected from the first speckle images or being associated with the first speckle images. The method further comprises determining first speckle contrast images based on the first speckle images, determining registered speckle contrast images by registering the first speckle contrast images based on the one or more transformation parameters and the image registration algorithm, and computing a weighted average of the registered first speckle contrast images.

Thus, a sequence of speckle contrast images may be registered or aligned without the use of a marker. The registered images may then be combined into a combined speckle contrast image, having a high resolution and accuracy The combined image may comprise information from a plurality of speckle images, e.g. a pixelwise average, preferably a weighted average, or the combined image can be a single most reliable image in e.g. a moving window of images, for instance the image with the smallest transformation (i.e., closest to the identity transformation using a suitable metric) relative to the subsequent image in a sequence of images.

This way, the images are less sensitive to noise due to motion. Motion can be caused by e.g. motion of the camera, in particular in handheld systems, or motion of the patient, due to e.g. muscle contractions. Thus, the embodiments in this disclosure may enable or improve speckle contrast imaging in a wide range of applications, including, for example, perfusion imaging large bowel tracts, requiring motion of the camera along the entire surface to be imaged, or perfusion imaging of skin burns, where a patient may be unable to remain motionless due to pain.

It is an advantage of the method that the method may be executed (essentially) in real-time using generally available hardware. In some embodiments, there may be a small delay based on, for example, a number of frames (e.g. 20 frames, or 20 frames of sufficient quality), a fixed amount of time (e.g. 1 second), or a time based on physiological properties (e.g. the time for one or two heartbeats or one or two respirations). Such a delay is generally not detrimental to clinical use. Physiological properties may be based on image analysis of the speckle images, the speckle contrast images, and/or the images from the plurality of images, on a predetermined constant based on knowledge on the physiological phenomena or may be based on external input, e.g. from a heart rate monitor.

It is another advantage of the method that the method does not need a fiducial marker to be placed in the field of view. This is especially relevant for areas where placing fiducial markers is undesirable, e.g. when imaging internal organs, burned tissue or brain tissue, or for relatively large image targets that would otherwise require a multitude of fiducial markers or repeatedly replacement of a marker.

As the method corrects and/or compensates for motion of the target area relative to the camera, more reliable perfusion images can be created. In some cases, reliable images may be acquired faster or more easily, for example when a target with irregular motion is imaged, and where an operator would otherwise have to wait for a period with little motion to acquire an image. For example, motion correction may comprise transforming one or more images based on detected apparent motion. Motion compensation may comprise combining a plurality of images, thus increasing the signal to noise ratio.

By registering the first speckle images or the first speckle contrast images, the plurality of first speckle images from the sequence of first speckle images may be used to determine a combined speckle contrast image with an increased contrast and/or spatial resolution, compared to the first speckle contrast images separately. The number of registered speckle images to be combined may depend on the clinical requirements and/or on quality parameters of the first speckle images.

Speckle contrast images may be computed based on the non-registered, and hence untransformed speckle images. Subsequently, the speckle contrast images may be transformed and then combined. The transformation may distort the speckle pattern or parts thereof, for example, speckles may be enlarged, shrunk, or deformed, especially for transformations that are more general than mere translations or rotations. Hence, computing speckle contrast images based on the untransformed speckle images may prevent introducing noise due to the transformation into the speckle contrast images.

Alternatively, the speckle images may be first registered, and hence transformed, and subsequently a speckle contrast image may be computed. This way, a temporal or spatio-temporal speckle contrast may be computed based on two or more registered speckle images. Using temporal or spatio-temporal speckle contrast may lead to a higher spatial resolution. In this embodiment, the images are preferably registered with sub-pixel accuracy.

It is a further advantage of this embodiment that only a single sequence of images is needed: the same sequence of image may be used for (determining transformation parameters for) image registration, for determining averaging weights, and for determining a combined laser speckle contrast image or perfusion image. However, in other embodiments, a second sequence of images may be used for determining the transformation parameters for image registration, and for determining the averaging weights.

In an embodiment, the method further comprises exposing the target area to second light of one or more second wavelengths, preferably coherent light of a second wavelength or light comprising a plurality of second wavelengths of the visible spectrum, wherein the exposure to the second light is alternated with the exposure to the first light or is simultaneous with the exposure to the first light. In this embodiment, the at least one sequence of images comprises a sequence of second images, the second images being captured during exposure with the second light; and the plurality of images is selected from the sequence of second images, each of the second images being associated with a first speckle image.

Thus, in an embodiment, the method of laser speckle contrast imaging comprises alternatingly or simultaneously exposing a target area to coherent first light of a first wavelength and to second light of one or more second wavelengths, preferably coherent light of a second wavelength or light comprising a plurality of second wavelengths of the visible spectrum, the target area preferably including living tissue. The method may further comprise capturing a sequence of first speckle images during the exposure with the first light and a sequence of second images during the exposure with the second light, a speckle image of the sequence of first speckle images being associated with an image of the sequence of second images. One or more transformation parameters of an registration algorithm for registering at least a part of the sequence of first speckle images may be determined based on a similarity measure of pixel values of groups of pixels in at least a part of the sequence of second images associated with the first speckle images. The method may further comprise determining registered first speckle images by registering the at least part of the sequence of first speckle images based on the one or more transformation parameters and the registration algorithm, and determining a combined speckle contrast image based on the registered first speckle images. Alternatively, the method may comprise determining first speckle contrast images based on the at least part of the sequence of first speckle images, determining registered speckle contrast images by registering the first speckle contrast images based on the one or more transformation parameters and the registration algorithm, and determining a combined speckle contrast image based on the registered first speckle contrast images.

In the first embodiment defined above, the first light and the second light are the same light with the same wavelength, and the sequence of first speckle images and the sequence of second images are the same sequence of images. However, in an advantageous embodiment, at least one of the one or more second wavelength is different from the first wavelength, and consequently, the second images are different from the first speckle images. A first speckle image and an associated second image can also be different parts of a single image. The images in the sequence of images may be frames in a video stream or in a multi-frame snapshot. In this disclosure, the second images may also be referred to as correction images. Typically, at least one group of pixels in each of the at least part of the sequence of second images is used to determine the one or more transformation parameters.

Thus, the first wavelength and the capturing of the sequence of first speckle images may be optimised for speckle contrast imaging (e.g. an optimised exposure time) in dependence of the quantity to be measured; while the one or more second wavelength and the capturing of the sequence of second images may be optimised to obtain images that can easily and accurately be registered, e.g. by ensuring a high contrast between anatomical features such as blood vessels and normal tissue. Typical combinations may be speckle images acquired using infrared light and second images acquired using white light, or speckle images acquired using red light and second images acquired using green or blue light, but of course, other combinations are also possible.

In an embodiment, the groups of pixels may represent predetermined features in the plurality of images, the predetermined features preferably being associated with objects, preferably anatomical structures, in the target area. Groups of pixels may be selected by a feature detection algorithm.

The features may be features associated with physical objects in the target area, e.g. features related to blood vessels, rather than e.g. image features not directly related to objects such as overexposed image parts or speckles. Predetermined features may be determined by e.g. belonging to a class of features, such as corners or regions with large differences in intensity. They may be further determined by e.g. a quality metric, restrictions on mutual distance between features, et cetera. In some embodiments, the neighbourhood of one or more determined features may be used to determine the displacement vectors and/or the transformation.

Determining a displacement based on a relatively small number of features, compared to the total number of pixels, may substantially reduce computation times, while still giving accurate results. This is especially the case for relatively simple motions, where e.g. the entire target area is displaced due to a motion of a camera.

In an embodiment, the method may further comprise filtering the plurality of images with a filter adapted to increase the probability that a group of pixels represents a feature corresponding to an anatomical feature. For example, a filter may determine overexposed and/or underexposed areas and/or other image artefacts, and may create a mask based on these areas or artefacts. Thus, determining features related to these areas or artefacts may be prevented.

In an embodiment, determining transformation parameters based on a similarity of pixel values of groups of pixels may comprise, for each group of pixels in an image from the plurality of images, determining a convolution or a cross-correlation with at least part of a different image from the plurality of images. The method may also comprise determining a polynomial expansion to model pixel values in pixel neighbourhoods in the plurality of images, comparing expansion coefficients, and determining displacement vectors based on the comparison.

In an embodiment, determining one or more transformation parameters may comprise determining a plurality of associated groups of pixels based on the similarity measure, each group of pixels belonging to a different image from the plurality of images, determining a plurality of displacement vectors based on positions of the groups of pixels relative to the respective images from the plurality of images, the displacement vectors representing motion of the target area relative to the image sensor, and determining the transformation parameters based on the plurality of displacement vectors.

By determining displacement vectors for a plurality of features, or pairs of corresponding features, arbitrary movements of the camera relative to the imaging target may be determined and corrected for. The displacement vectors may be used to determine e.g. an affine transformation, a projective transformation, or a homography, which may correct for e.g. translation, rotation, scaling and shearing of an image based on image data alone. Thus, the method does not require information about e.g. distance between camera and target, incident angle, et cetera.

The determination of displacement vectors and/or the determination of the one or more transformations may be based on optical flow parameters, determined using a suitable optical flow algorithm, e.g. a dense optical flow algorithm or a sparse optical flow algorithm.

In an embodiment, determining a combined speckle contrast image may further comprise computing an average of the registered first speckle images, respectively of the registered first speckle contrast images, the average preferably being a weighted average, a weight of an image preferably being based on the transformation parameters or based on a relative magnitude of the speckle contrast. Alternatively, combining speckle images or speckle contrast images may comprise filtering the at least part of the first speckle images, respectively first speckle contrast images, with e.g. a median filter or a minimum or maximum filter, et cetera. Weights for weighted averaging may be based e.g. on a quantity derived from the speckle contrast or derived from the displacement vectors.

In an embodiment, computing a combined laser speckle contrast image may comprise computing an average, preferably a weighted average, of the registered sequence of speckle images.

In an embodiment, the method may further comprise, for each first speckle image or each first speckle contrast image associated with an image from the plurality of images, determining a transformation size associated with the respective first speckle image or first speckle contrast image based on the plurality of displacement vectors, preferably based on the lengths of the plurality of displacement vectors, and/or on parameters defining the determined transformation. The weighted average may be determined using weights based on the determined transformation size associated with the respective first speckle contrast image, preferably the weight being inversely correlated to the determined transformation size.

A weight based on the size or amount of displacement, or on the size or amount of transformation may be determined quickly for each image, independent of other images. The transformation size may e.g. be based on a norm of a matrix representing the transformation, or the norm of matrix representing a difference between the transformation and the identity transformation. The transformation size may also be based on e.g. a statistically representative measure of the displacement vectors, e.g., the average, median, n-th percentile, or maximum displacement vector length. Images with a large amount of displacement are generally noisier, and may therefore be assigned a lower weight, thus increasing the quality of the combined image.

In an embodiment, the method may further comprise determining, for each first speckle image, a normalised amount of speckle contrast or an amount of change in speckle contrast relative to one or more previous and/or subsequent images in the sequence of first speckle contrast images. The weighted average may be determined using weights based on the determined normalised amount of speckle contrast or the determined change in speckle contrast associated with the respective first speckle contrast image. Alternatively, or additionally, weights may be determined based on a normalised amount of speckle contrast or an amount of change in speckle contrast for the second speckle contrast images.

Weights based on differences or changes in speckle contrast, especially sudden changes, may be indicative for image quality. Typically, speckle contrast, and hence these weights, may be affected by various factors in the entire system, e.g. motion of the camera relative to the target area, movement of fibres or other factors influencing the optical path length or fluctuating lighting conditions. Hence, using weights based on speckle contrast, a higher quality combined image may be obtained. Typically, speckle contrast is determined in arbitrary units, so weights may be determined by analysing a sequence of speckle images. As speckle contrast is inversely correlated with perfusion, speckle contrast-based perfusion units could similarly be used.

In an embodiment, the algorithm may be applied to a predefined region of interest in a field of view of a camera. Such a region of interest may be determined by a user, or may be predetermined. For example, the outer border of the images may be ignored. and/or hidden from view, e.g. to prevent a transformed image border from being visible. Applying the algorithm, or part of the algorithm, to only part of an image may be faster. The region of interest may be transformed based on the determined transformations. In a different embodiment, the algorithm may be applied to the entire image.

In an embodiment, the plurality of images may be the sequence of first speckle images. In such an embodiment, the first wavelength is preferably a wavelength in the green or the blue part of the electromagnetic spectrum. This way, a balance may be struck between a good speckle signal and good visual distinctiveness (i.e., a high contrast of anatomical features, which is to be differentiated from a high speckle contrast), which is advantageous for determining features. Thus, there is no pre-processing step required to increase the contrast of the speckle image.

Alternatively, a first wavelength in the red part of the electromagnetic spectrum may be used, preferably in the range 600-700 nm, more preferably in the range 620-660 nm, or in the infrared part of the electromagnetic spectrum, preferably in the range 700-1200 nm. Depending on the tissue type and imaging parameters such as exposure time, the visual distinctiveness may be sufficient for adequate determination of features. Since red light and infrared light is mostly reflected by red blood cells, these wavelengths result in speckles with a relatively high intensity and are thus very suitable for speckle contrast imaging of blood flow.

As in these embodiments, the first speckle images and the images from the plurality of images are the same images, the images may be acquired with a relatively simple system, requiring only a single light source and a single camera.

In an embodiment, the light of the at least second wavelength may be light of at least a second wavelength different from the first wavelength, preferably coherent light of a predetermined second wavelength, preferably in the green or blue part of the electromagnetic spectrum, preferably in the range 380-590 nm, more preferably in the range 470-570 nm, even more preferably in the range 520-560 nm. Blue or, especially, green light may result in a high contrast or visual distinctiveness, as it is absorbed in the blood vessels much more strongly than by normal tissue. Thus, features, such edges or corners, related to blood vessels may be used to determine the transformation parameters,

As the first speckle images themselves are inherently noisy (as far as imaging of anatomical features is concerned), it can be preferable to use second images based on a different wavelength to determine displacement vectors. This way, the first speckle images may be acquired based on light selected to optimise the speckle contrast signal, while the second images may be acquired based on light selected to optimise visual distinctiveness. Such a system is particularly advantageous for imaging tissues where the blood perfusion is relatively deep, e.g. the skin. In such tissues, most of the green or blue light does not penetrate deep enough to interact with the blood cells, resulting in a relatively noise free image.

In an embodiment, the first wavelength is preferably a wavelength in the red part of the electromagnetic spectrum, preferably in the range 600-700 nm, more preferably in the range 620-660 nm, or in the infrared part of the electromagnetic spectrum, preferably in the range 700-1200 nm. Red light and infrared light is mostly reflected by red blood cells, making it very suitable for speckle contrast imaging of blood flow. Infrared light has a larger penetration depth than red light. Red light may be easier to integrate into existing systems, using e.g. a red channel of an RGB camera to acquire a speckle image.

Preferably, the first wavelength is selected to be scattered or reflected by the fluid of interest; for example, red or near-infrared light may be used for imaging blood in blood vessels. Preferably, the first wavelength may be selected based on the required penetration depth. Light with a relatively high penetration depth may allow light scattered by the bodily fluid of interest to be detected with a sufficient signal to noise ratio even at some depth in the imaged tissue.

Preferably, the second wavelength is selected to provide an image with a high visual distinctiveness resulting in consistent features on the tissue surface in the image. For example, green light may be used for imaging blood vessels in internal organs, as green light typically is absorbed much more strongly by blood than by tissues. The light of the second wavelength can be either coherent or incoherent light. The second images may also be based on a multitude of wavelengths, e.g. white light may be used. The light of the second wavelength may be generated by e.g. a second coherent light source. Alternatively, light of the first wavelength and the light of the second wavelength may be generated by a single coherent light source configured to generate coherent light at a plurality of wavelengths.

In an embodiment, the sequence of second images may be a sequence of second speckle images and the method may further comprise determining second speckle contrast images based on the sequence of second speckle images and adjusting or correcting the first speckle contrast images based on changes in speckle contrast magnitude in the sequence of second speckle contrast images.

Multi-spectral coherent correction, also called dual laser correction, may remove or reduce noise in the first speckle contrast images by adjusting the determined speckle contrast in the first speckle images based on a change in determined speckle contrast in sequence of second images. The adjustment may be based on a predetermined correlation between the speckle contrast of the first speckle contrast images and the speckle contrast of the second speckle contrast images.

Multi-spectral coherent correction may advantageously be combined with image registration using second images by using the second images based on the second wavelength both for multi-spectral coherent correction and for image registration. In such an embodiment, the second wavelength preferably has a relatively small penetration depth. This way the second image may comprise information that mostly relates to the surface of the target area. This is especially true for tissues with little perfusion close to the surface, such as the skin, scar tissue, and some tumour types.

In an embodiment, the method may further comprise dividing each image in the at least part of the sequence of first speckle images, respectively first speckle contrast images, and each image in the plurality of images into a plurality of regions, preferably disjoint regions. Preferably, the regions in the image from the plurality of images correspond to the regions in the associated first speckle image, respectively first speckle contrast image. Determining transformation parameters may comprise determining transformation parameters for each region, and determining a sequence of registered first speckle images, respectively first speckle contrast images, may comprise registering each region of the first speckle image, respectively first speckle contrast image, based on the transformation based on the corresponding region in the image from the plurality of images.

The regions may be determined based on e.g. the geometry of the image, e.g. a grid of rectangular or triangular regions, or based on image properties, e.g. light intensity or groups of pixels that appear to belong to an anatomical structure.

This way, local movements in part of the image may be corrected, leading to a higher quality combined image. Local movements are typically caused by motion in the target, e.g. due to the person moving, respiration, heartbeat, or muscle contraction such as peristaltic motion in the lower abdomen. The regions may be as small as single pixels. If a weighted average is used to combine two or more images, weights may be assigned to each region separately and/or to the image as a whole. Combining images may likewise be region based, or be done on an image-by-image basis.

In an embodiment, the target area may comprise a perfused organ, preferably perfused by a bodily fluid, more preferably perfused by blood and/or lymph fluid, and/or may comprise one or more blood vessels and/or lymphatic vessels. The method may further comprise computing a perfusion intensity, preferably a blood perfusion intensity or a lymph perfusion intensity, based on the combined speckle image.

The method may further include post-processing the images, e.g. thresholding, false colouring, overlying on other images, e.g. white light images, and/or displaying the combined image or a derivative thereof.

In a second aspect, the invention is further related to a hardware module for an imaging device, preferably a medical imaging device, comprising a first light source a first light source for exposing a target area to coherent first light of a first wavelength, the target area preferably including living tissue. The hardware module may further comprise an image sensor system with one or more image sensors for capturing at least one sequence of images, the at least one sequence of images comprising first speckle images, the first speckle images being captured during the exposure with the first light. The hardware module may further comprise a computer readable storage medium having computer readable program code embodied therewith, and a processor, preferably a microprocessor, more preferably a graphics processing unit, coupled to the computer readable storage medium, wherein responsive to executing the computer readable program code, the processor is configured to: determine one or more transformation parameters of an image registration algorithm for registering the first speckle images with each other, the transformation parameters being based on a similarity measure of pixel values of at least one group of pixels in each of a plurality of images, the images in the plurality of images being selected from the first laser speckle images or being associated with the first speckle images, the transformation parameters preferably defining one of: a homography, a projective transformation, or an affine transformation; and determine first speckle contrast images based on the first speckle images, determine registered speckle contrast images by registering the first speckle contrast images based on the one or more transformation parameters and the image registration algorithm, and compute a weighted average of the registered first speckle contrast images.

In an embodiment, the hardware module may comprise a second light source for illuminating, simultaneous or alternatingly with the first light source, the target area with light of at least a second wavelength, different from the first wavelength. The at least one image sensor may be configured to capture a sequence of second images, the second images being captured during exposure with the second light. The plurality of images may be selected from the sequence of second images, each of the second images being associated with a first speckle image.

The image sensor system may comprise a first image sensor for capturing the sequence of first images, and a second image sensor for capturing the sequence of second images, or a single image sensor for capturing both the sequence of first images and the sequence of second images.

In an embodiment, the hardware module may further comprise a display for displaying the combined speckle image and/or a derivative thereof, preferably a perfusion intensity image. Alternatively or additionally, the hardware module may comprise a video output for outputting the combined speckle image and/or the derivative thereof.

The image sensor system may comprise a first image sensor for capturing images of the first wavelength and a second image sensor for capturing images of the at least second wavelength. The first image sensor and the second image sensor may be the same image sensor, different parts of a single image sensor, e.g. red and green channels from an RGB camera, or different image sensors. The module may further comprise optics to guide light from the first light source and from the optional second light source to a target area and/or to guide light from the target area to the first and second image sensors.

The invention is further related to a medical imaging device, preferably a endoscope, a laparoscope, a surgical robot, a handheld laser speckle contrast imaging device or an open surgical laser speckle contrast imaging system comprising such a hardware module.

In a further aspect, the invention is related to a computation module for a laser speckle imaging system, comprising a computer readable storage medium having at least part of a program embodied therewith, and a processor, preferably a microprocessor, more preferably a graphics processing unit, coupled to the computer readable storage medium, wherein responsive to executing the computer readable storage code, the processor is configured to perform executable operations. The executable operations may comprise:

receiving at least one sequence of images, the at least one sequence of images comprising first speckle images, the first speckle images having been captured during exposure of a target area to coherent first light of a first wavelength, the target area including living tissue; determining one or more transformation parameters of an image registration algorithm for registering the first speckle images with each other, the transformation parameters being based on a similarity measure of pixel values of at least one group of pixels in each of a plurality of images of the at least one sequence of images, the images in the plurality of images being selected from the first speckle images or being associated with the first speckle images, the transformation parameters preferably defining one of: a homography, a projective transformation, or an affine transformation; and determining first speckle contrast images based on the first speckle images, determining registered speckle contrast images by registering the first speckle contrast images based on the one or more transformation parameters and the image registration algorithm, and compute a weighted average of the registered first speckle contrast images.

Such a computation module may e.g. be added to an existing or new medical imaging device such as a laparoscope or an endoscope, in order to improve laser speckle contrast imaging, in particular perfusion imaging. In an embodiment, the method steps described in this disclosure may be executed by a processor in a device for coupling coherent light into an endoscopic system. Such a device may be coupled between a light source and a video processor of an endoscopic system, and an endoscope, e.g. a laparoscope, of the endoscopic system. The coupling device may thus add laser speckle imaging capabilities to an endoscopic system. Such a coupling device has been described in more detail in Dutch patent application NL 2026240.

In an embodiment, the at least one sequence of images comprises a sequence of second images, the second images being captured during exposure with second light, the second light having one or more second wavelengths, preferably the second light being coherent light of a second wavelength or the second light comprising a plurality of second wavelengths of the visible spectrum, wherein the exposure to the second light is alternated with the exposure to the first light or is simultaneous with the exposure to the first light. The plurality of images may be selected from the sequence of second images, each of the second images being associated with a first speckle image.

The invention may also relate to a computer program or suite of computer programs comprising at least one software code portion or a computer program product storing at least one software code portion, the software code portion, when run on a computer system, being configured for executing any of the method steps described above.

The invention may further relate to a non-transitory computer-readable storage medium storing at least one software code portion, the software code portion, when executed or processed by a computer, is configured to perform any of the method steps as described above.

The invention will be further illustrated with reference to the attached drawings, which schematically will show embodiments according to the invention. It will be understood that the invention is not in any way restricted to these specific embodiments.

Brief description of the drawings

The following description of the figures of specific embodiments of the invention is merely exemplary in nature and is not intended to limit the present teachings, their application or uses.

Fig. 1A schematically depicts a system for motion-compensated laser speckle contrast imaging according to an embodiment of the invention and Fig. 1B-D depict flow diagrams for laser speckle contrast imaging according to embodiments of the invention;

Fig. 2A-C depict a raw speckle image, a laser speckle contrast image based on the raw speckle image, and a perfusion image based on the laser speckle contrast image;

Fig. 3A and 3B depict flow diagrams for laser speckle contrast imaging according to embodiments of the invention;

**Fig. 4A** and **4B** depict flow diagrams for laser speckle contrast imaging according to embodiments of the invention;

**Fig. 5** depicts a method for determining a transformation according to an embodiment of the invention;

Fig. 6A-D depict methods for determining a transformation according to an embodiment of the invention;

**Fig. 7A** and **7B** depict flow diagrams for laser speckle contrast imaging combining more than two raw speckle images according to an embodiment of the invention;

**Fig. 8** depicts a flow diagram for computing a corrected laser speckle contrast image according to an embodiment of the invention;

**Fig. 9** schematically depicts determining motion compensate speckle contrast image based on a weighted average, according to an embodiment of the invention; and

**Fig. 10** is a block diagram illustrating an exemplary data processing system that may be used for executing methods and software products described in this application.

Detailed description

Laser speckle contrast images may be based on spatial contrast, temporal contrast, or a combination. In general, using spatial contrast leads to a high temporal resolution but a relatively low spatial resolution. Additionally, individual images may suffer from e.g. quality loss due to motion or lighting artefacts, resulting in an image quality that may vary from image to image. On the other hand, using a temporal contrast is associated with a relatively high spatial resolution and a relatively low temporal resolution. However, the quality of temporal contrast may be strongly affected by motion of the target relative to the camera, which may lead to pixels being incorrectly combined. Mixed methods may share some advantages and disadvantages of both methods.

In this disclosure, speckle images may also be referred to as raw speckle images to better differentiate between (raw) speckle images and speckle contrast images. The term 'raw speckle image' may thus refer to an image representing a speckle pattern, with pixels having pixels values representing a light intensity. Raw speckle images may be unprocessed images or (pre-)processed images. The term 'speckle contrast image' may be used to refer to a processed speckle image with pixels having pixel values representing a speckle contrast magnitude, typically a relative standard deviation over a predefined neighbourhood of the pixel.

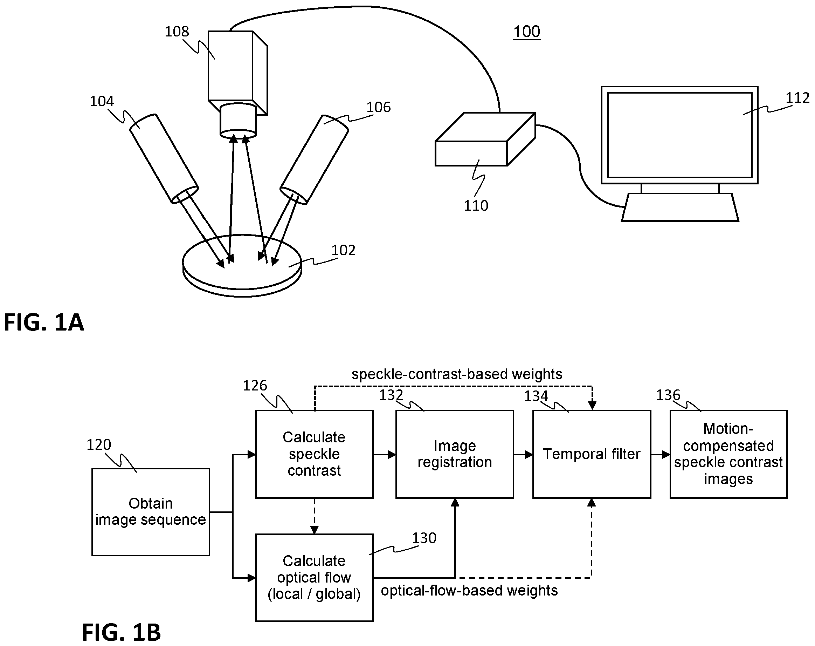

**Fig. 1A** schematically depicts a system **100** for motion-compensated laser speckle contrast imaging according to an embodiment of the invention. The system may comprise a first light source **104** for generating coherent light, e.g. laser light, of a first wavelength for illuminating a target area **102.** The target is preferably living tissue, e.g. skin, bowel, or brain tissue. The first wavelength may be selected to interact with a bodily fluid which may move through the target, for instance blood or lymph fluid. The first wavelength may be in the red or (near) infrared part of the electromagnetic spectrum, e.g. in the range 600-700 nm, preferably in the range 620-660 nm, or in the range 700-1200 nm. The first wavelength may be selected based on the bodily fluid of interest and/or the tissue being imaged. The first wavelength may also be selected based on the properties of an imaging sensor. Depending on, inter alia, exposure time, size of the imaged area, speckle size, and image resolution, different quantities of interest may be imaged, e.g. flow in individual large or small blood vessels, or microvascular perfusion of a target area.

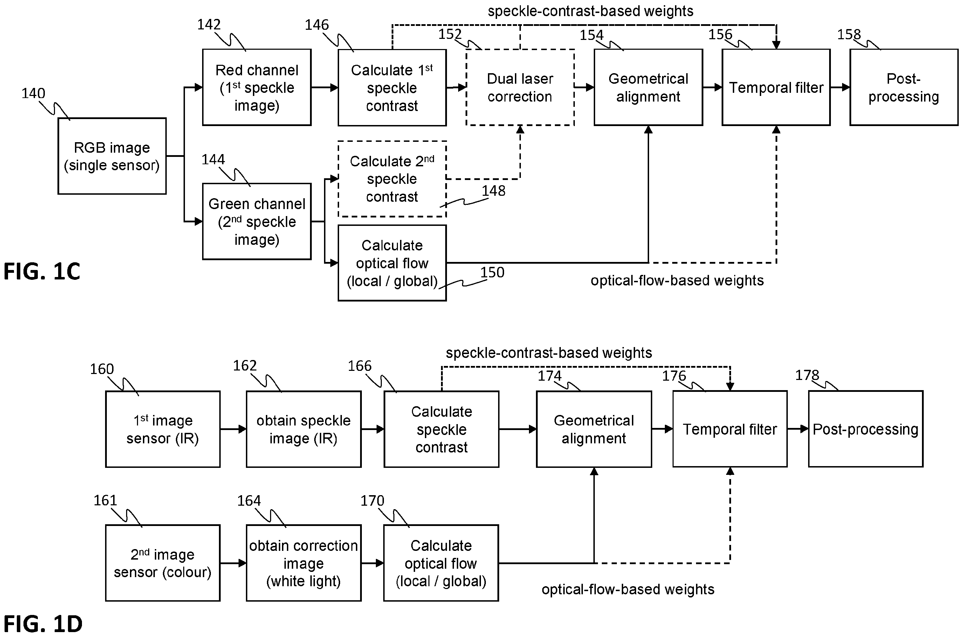

In an embodiment, the system may further comprise a second light source **106** for generating light of at least a second wavelength, preferably comprising light of the green part of the electromagnetic spectrum, for illuminating the target area **102.** The at least second wavelength may be selected to comprise a wavelength that creates images with a high visual distinctiveness, that is, a high contrast of anatomical features. The second wavelength may be selected based on the tissue in the target area. In general, the light of the at least second wavelength may be coherent light or incoherent light, and may be monochromatic, e.g. blue or green narrow-band imaging light, or polychromatic light, e.g. white light. In other embodiments, only the first light source is used. In the embodiment depicted in **Fig. 1C**, the light of the at least second wavelength is monochromatic coherent light. The second wavelength may be generated by e.g. a second coherent light source. Alternatively, light of the first wavelength and the light of the second wavelength may be generated by a single coherent light source configured to generate coherent light at a plurality of wavelengths.

The system may further comprise one or more image sensors **108** for capturing images associated with light of the first wavelength and, when applicable, images associated with light of the at least second wavelength, the light of the first and at least second wavelengths having interacted with the target in the target area. In a different embodiment, the system may comprise a plurality of cameras, for example a first camera for acquiring first raw speckle images associated with the first wavelength and a second camera for acquiring correction images associated with the second wavelength. The system may furthermore comprise additional optics, e.g. optical fibres, lenses, or beam splitters, to guide light from the one or more light sources to the target area and from the target area to the one or more image sensors.

When the target is illuminated with coherent light, a laser speckle pattern may be formed through self-interference. The images may be received and processed by a processing unit **110.** Examples will be described in further detail with reference to **Fig. 1B-D**. The processing unit may output processed images in essentially real-time to e.g. a display or to a computer **112.** The processing unit may be a separate unit or may be part of the computer. The processed images may be displayed by the display or computer.

In an embodiment, an endoscope or laparoscope may be used to guide light to the target area and to acquire images. The one or more light sources, the one or more image sensors, the processing unit and the display may e.g. be part of an endoscopic system.

**Fig. 1B-D** depict a flow diagrams for motion-compensated laser speckle contrast imaging according to an embodiments of the invention. Alternatives that are mentioned with respect to one of these embodiments can also be applied to the other embodiments, unless where such combination would result in a contradictory description.

**Fig. 1B** depicts a flow diagram for motion-compensated laser speckle contrast imaging according to an embodiment of the invention. In this embodiment, only coherent light of the first wavelength generated by the first light source **104** is used, which can be e.g. green or, preferably, red light. The one or more image sensors **108** is a single image sensor, typically a monochromatic image sensor optimised or at least suitable for the used wavelength. As was indicated above and will be shown in the examples of **Fig. 1C** and **1D** below, other embodiments may use different configurations.

In a first step **120**, a sequence of raw speckle images is obtained, e.g. captured or received from an external source. These speckle images will also be used as correction images. Based on each first raw speckle image, a first speckle contrast image may be computed **126**. Speckle contrast may be determined in any suitable way, e.g. by a convolution with a predetermined convolution kernel, or by determining the relative standard deviation of pixel value intensities in a sliding window. As the speckle contrast is correlated with perfusion, perfusion unit weights may be determined based on the speckle contrast values of the first speckle contrast images.

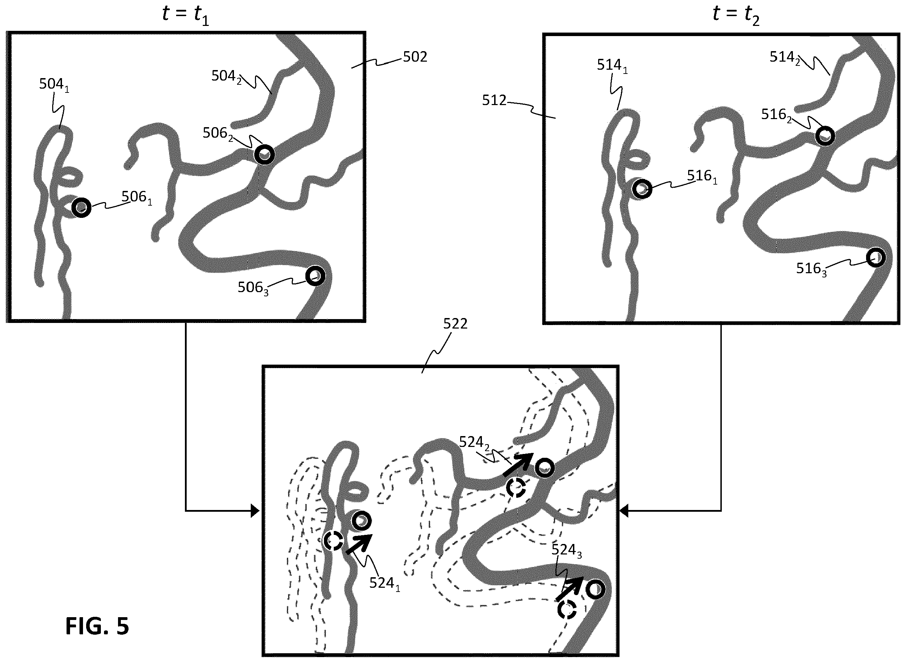

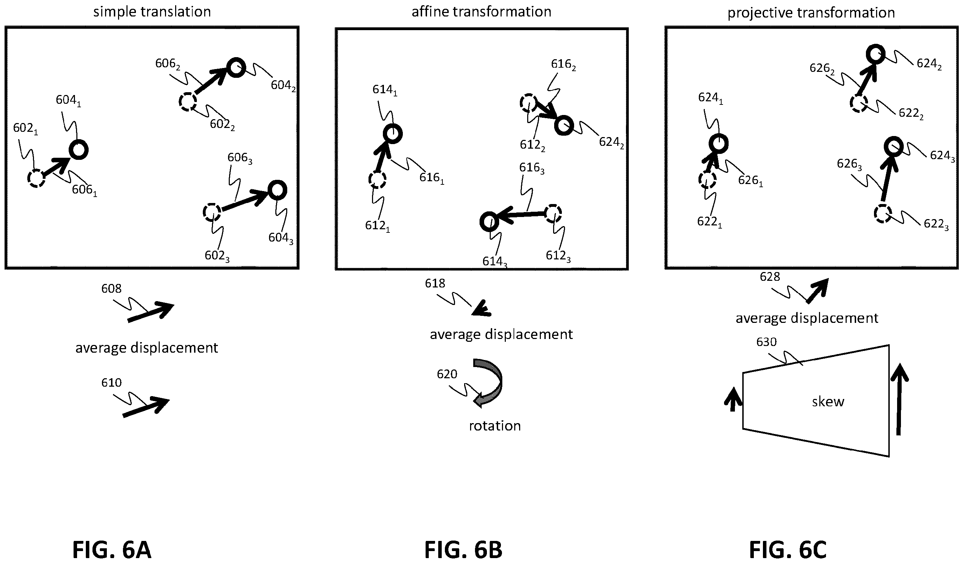

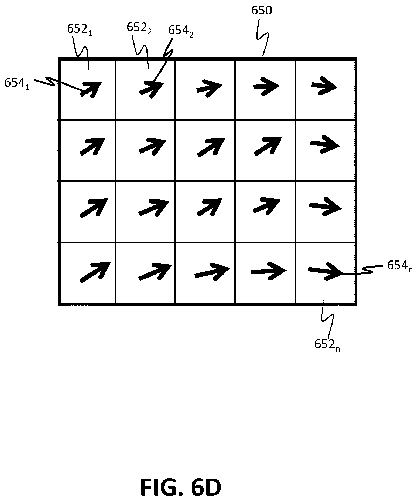

In a single-wavelength embodiment, either the raw speckle images or the speckle contrast images may be used as correction images. In each correction image, positions of predetermined object features may be determined **130**. Based on the positions of the predetermined object features in two or more correction images, displacement vectors identifying motion of the target area may be determined, for example using an optical flow algorithm. This will be described in more detail with reference to **Fig. 5** and **6**. A (sparse) optical flow algorithm may be used to determine displacement vectors based on selected features. Other embodiments may use e.g. a dense optical flow algorithm; in that case, there is no need to determine features.

Based on the displacement vectors, transformations for registering images in the second sequence of images may be determined. Preferably, the transformations are selected from the class of homographies, from the class of projective transformations, or from the class of affine transformations. Optionally, optical flow weights may be determined based on the displacement vectors or on parameters defining the transformation.

The determined transformations may then be used to register **134**, or geometrically align, the speckle contrast images with each other, resulting in registered first speckle contrast images. In an alternative embodiment, the raw speckle images may be registered before computing the speckle contrast. However, as the image registration may affect the pixel values and hence the contrast, such an embodiment is less preferred.

Subsequently, the registered first speckle contrast images may be combined **136**, e.g., using a temporal filter. The temporal filter may comprise averaging a plurality of first speckle contrast images. The averaging may be weighted averaging, with the weights being based on the optical flow weights and/or based on perfusion unit weights. In an alternative embodiment, the registered raw speckle images may be combined using the temporal filter, and a speckle contrast image may be determined based on the combine draw speckle image. This results in motion-compensated speckle contrast images

Afterwards, the combined first raw speckle images may be post-processed, e.g. converted to perfusion values, thresholded to indicate areas with high or low perfusion, overlain on a white light image of the target area, et cetera. The post-processing may be done by e.g. the processing unit **110** or the computer **112.Fig. 1C** depicts a flow diagram for motion-compensated laser speckle contrast imaging according to an embodiment of the invention. In the depicted embodiment, the light of the first wavelength generated by the first light source **104** is red light, and the light of the at least second wavelength generated by the second light source **106** is coherent green light. The one or more image sensors **108** are a single sensor comprising red, green, and blue channels. The first wavelength and the second wavelength are selected to minimise crosstalk. The signal in the red channel caused by the green light is substantially smaller than the signal caused by the red light; and the signal in the green channel caused by the red light is substantially smaller than the signal caused by the green light. As was indicated above, other embodiments may use different configurations.

In a first step **140**, a sequence of RGB images is received. A first sequence of first raw speckle images may be extracted **142** from the red channel of sequence of the RGB image, and a second sequence of correction images may be extracted **144** from the green channel of the RGB image. The sequence of correction images may be a second sequence of second raw speckle images. Each first raw speckle image may be associated with the correction image extracted from the same RGB image. In other embodiments, the first raw speckle images and the correction images may be acquired by different cameras, by different sensors of a multi-sensor camera (e.g., a 3CCD camera), by other (colour) channels of a single camera (e.g., a YUV camera), or, if the target is illuminated alternately with light of the first wavelength and light of the at least second wavelength, the images may be acquired alternately by a single monochrome camera.

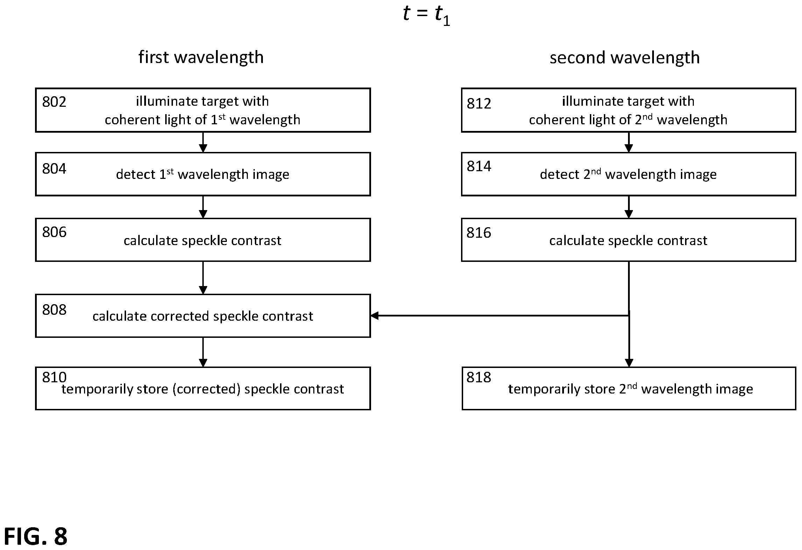

Based on each first raw speckle image, a first speckle contrast image may be computed **146**. Optionally, based on each second raw speckle image, a second speckle contrast image may be computed **148**. Speckle contrast may be determined in any suitable way, e.g. by a convolution with a predetermined convolution kernel, or by determining the relative standard deviation of pixel value intensities in a sliding window. As the speckle contrast is correlated with perfusion, perfusion unit weights may be determined based on the speckle contrast values of the first speckle contrast images. The second speckle contrast image may optionally be used to correct **152** the first speckle contrast image, as will be described in more detail with reference to **Fig. 8.** In that case, the corrected speckle contrast values may be used to determine perfusion unit weights.

In each correction image (i.e., in this embodiment, in each second raw speckle image), positions of predetermined object features may be determined **150**. Based on the positions of the predetermined object features in two or more correction images, displacement vectors identifying motion of the target area may be determined, for example using an optical flow algorithm. This will be described in more detail with reference to **Fig. 5** and **6.** A (sparse) optical flow algorithm may be used to determine displacement vectors based on selected features. Other embodiments may use e.g. a dense optical flow algorithm; in that case, there is no need to determine features.

Based on the displacement vectors, transformations for registering images in the second sequence of images may be determined. Preferably, the transformations are selected from the class of homographies, from the class of projective transformations, or from the class of affine transformations. Optionally, optical flow weights may be determined based on the displacement vectors or on parameters defining the transformation.

The determined transformations may then be used to register **154**, or geometrically align, the first speckle contrast images associated with the correction images.

Subsequently, the registered first speckle contrast images may be combined **156**, e.g. using a temporal filter. The temporal filter may comprise averaging a plurality of first speckle contrast images. The averaging may be weighted averaging, with the weights being based on the optical flow weights and/or based on perfusion unit weights.

In a final step **158**, the combined first raw speckle images may be post-processed, e.g. converted to perfusion values, thresholded to indicate areas with high or low perfusion, overlain on a white light image of the target area, et cetera. The post-processing may be done by e.g. the processing unit **110** or the computer **112.**

**Fig. 1D** depicts a flow diagram for motion-compensated laser speckle contrast imaging according to an embodiment of the invention. In the depicted embodiment, the light of the first wavelength generated by the first light source **104** is infrared light, but other colours such as red, green, or blue are also possible. The light of the at least second wavelength generated by the second light source **106** is (incoherent) white light. An advantage of using infrared light and white light is that both may be used simultaneously without substantially affecting each other

As depicted, the one or more image sensors **108** are two image sensors in two cameras, an infrared camera and a colour camera. This can be practical if the laser speckle imaging is added to a system already comprising a colour camera, for instance in an open surgery setting. Additionally, having dedicated image sensors may allow separate optimisation of hardware and/or equipment parameters such as exposure time. As was indicated above, other embodiments may use different configurations. For example, in some embodiments, a single camera may be used to capture both the infrared and the (white light) colour images. An advantage of using a single camera is that the infrared images and the colour images are automatically aligned and associated with each other.

In a first step **160**, a first sequence of first images is captured by the infrared camera. This first sequence may be stored by the image processor as a sequence of raw speckle images. In a second step **161**, a second sequence of second images is captured by the colour camera. This second sequence may be stored **164** as a sequence of correction images. Each raw speckle image may be associated with one or more correction images. Preferably, each raw speckle image is associated at least with the correction image that was captured closest in time to, preferably simultaneous with the raw speckle image. Preferably, the frame rates of the first and second cameras are chosen such as to allow a straightforward association, e.g. by selecting one frame rate as a integer multiple of the other frame rate.

Based on each raw speckle image, a speckle contrast image may be computed **166**. Speckle contrast may be determined in any suitable way, e.g. by a convolution with a predetermined convolution kernel, or by determining the relative standard deviation of pixel value intensities in a sliding window. As the speckle contrast is correlated with perfusion, perfusion unit weights may be determined based on the speckle contrast values of the first speckle contrast images.

In each correction image (i.e., in this embodiment, in each white light image), positions of predetermined object features may be determined **170**. Based on the positions of the predetermined object features in two or more correction images, displacement vectors identifying motion of the target area may be determined, for example using an optical flow algorithm. This will be described in more detail with reference to **Fig. 5** and **6.** A (sparse) optical flow algorithm may be used to determine displacement vectors based on selected features. Other embodiments may use e.g. a dense optical flow algorithm; in that case, there is no need to determine features.

Based on the displacement vectors, transformations for registering images in the sequence of correction images may be determined. Preferably, the transformations are selected from the class of homographies, from the class of projective transformations, or from the class of affine transformations. Optionally, optical flow weights may be determined based on the displacement vectors or on parameters defining the transformation.

The determined transformations may then be used to register **174**, or geometrically align, the first speckle contrast images associated with the correction images. Since, in this embodiment, two cameras are used, the two cameras do not share a field of view and frame of reference. Consequently, registering the speckle contrast images based on the transformation parameters determined using the correction images may comprise applying a transformation to the transformation parameters to account for this change in frame of reference. If the cameras are positioned in a fixed position relative to each other, this transformation may be predetermined. Otherwise, the transformation can be determined based on e.g. image processing of calibration images or markers. Markers can be either natural or artificial.

Subsequently, the registered first speckle contrast images may be combined **176**, e.g. using a temporal filter. The temporal filter may comprise averaging a plurality of first speckle contrast images. The averaging may be weighted averaging, with the weights being based on the optical flow weights and/or based on perfusion unit weights.

In a final step **178**, the combined first raw speckle images may be post-processed, e.g. converted to perfusion values, thresholded to indicate areas with high or low perfusion, overlain on a white light image of the target area, et cetera. The post-processing may be done by e.g. the processing unit **110** or the computer **112.**

In an embodiment, the method steps described in this disclosure may be executed by a processor in a device for coupling coherent light into an endoscopic system. Such a device may be coupled between a light source and a video processor of an endoscopic system, and an endoscope, e.g. a laparoscope, of the endoscopic system. The coupling device may thus add laser speckle imaging capabilities to an endoscopic system. Such a coupling device has been described in more detail in Dutch patent application NL 2026240.

In an alternative embodiment, the method steps described in this disclosure may be applied in an open surgical setting, possibly in combination with a pre-existing imaging system.

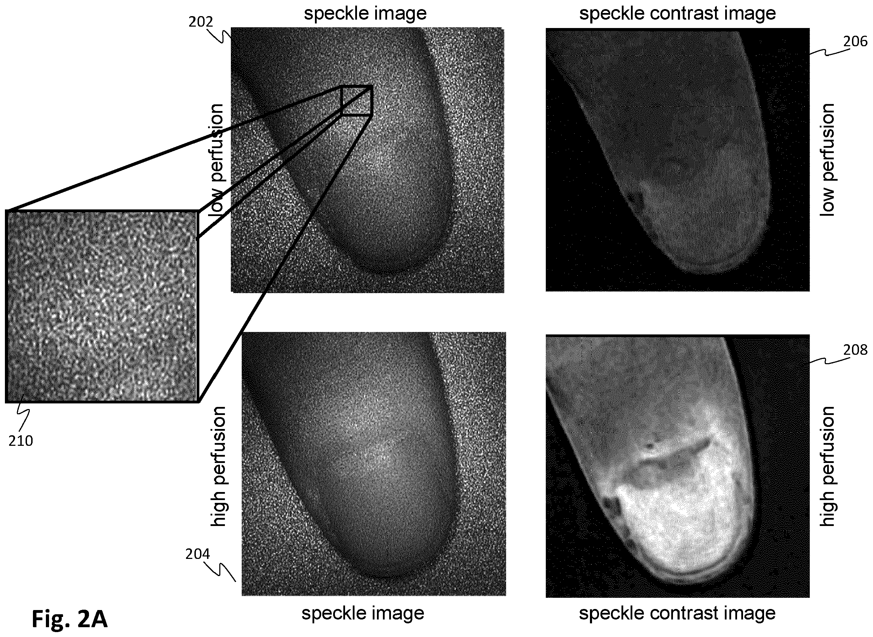

**Fig. 2A** depicts a raw speckle images, and laser speckle contrast images based on the raw speckle images, of a target with low perfusion and a target with high perfusion. Images **202** and **204** are raw speckle images of the tip of a human finger, including a nail bed. When image **202** was obtained, blood flow through the finger was restricted, resulting in a low blood perfusion of the finger (artificial ischemia). When image **204** was obtained, blood flow was unrestricted, resulting in a much higher blood perfusion, compared to the previous situation. For a human viewer, it is difficult to see differences in the speckle pattern associated with the difference in perfusion. A zoomed-in part **210** of image **204** is also shown, displaying the speckle structure in more detail.

Images **206** and **208** are speckle contrast images based on images **202** and **204,** respectively. A light colour represents a low contrast, and hence a high perfusion, while a dark colour represents a high contrast, and hence a low perfusion. In these images, the difference in perfusion is immediately clear, especially in the nail bed where the blood flow occurs relatively close to the surface.

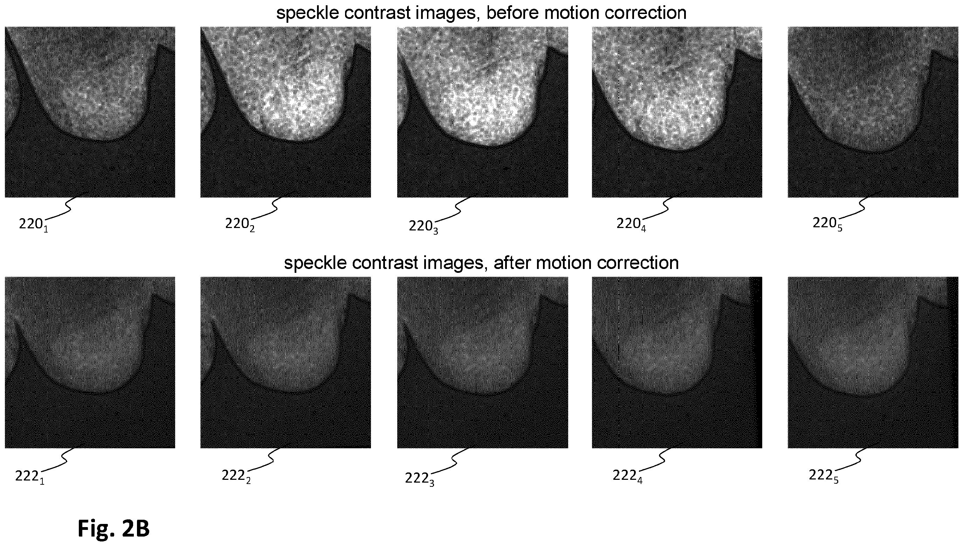

**Fig. 2B** depicts a series of laser speckle contrast images of a low perfusion target exhibiting motion, before motion correction and after motion correction according to an embodiment of the invention. Images $220_{1-5}$ are speckle contrast images of the tip of a human finger, including a nail bed. During the acquisition of images $220_{2-4}$, the finger moved, leading to loss of contrast due to finger motion. If a user is interested in blood flow, the low speckle contrast in images $220_{2-4}$, represented by a light colour, may be considered a motion artefact. Images $222_{1-5}$ are based on the same raw speckle images as images $220_{1-5}$, respectively, but have been corrected by a motion correction algorithm according to an embodiment of the invention.

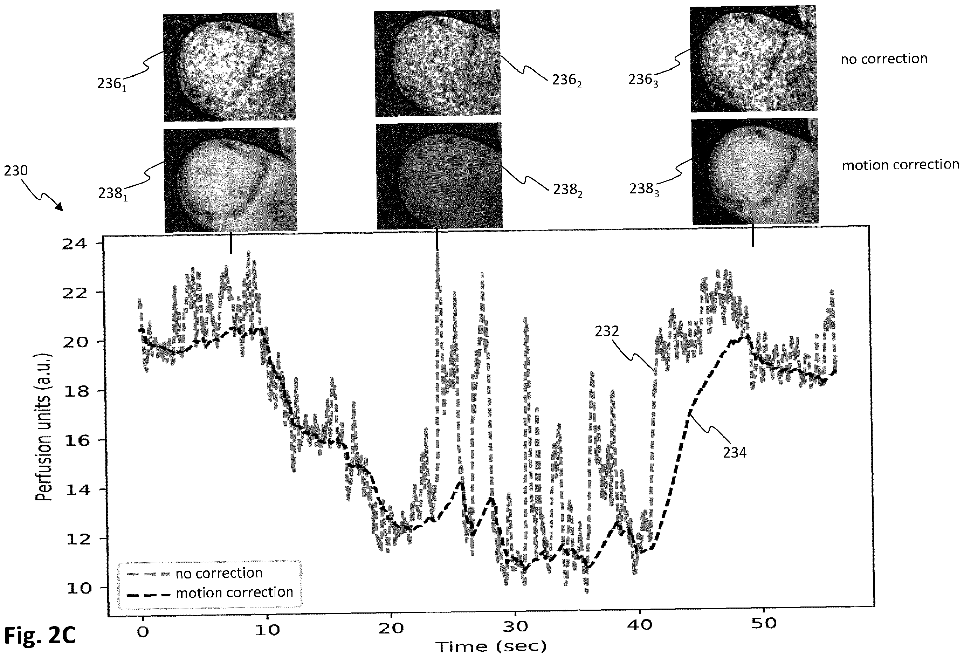

**Fig. 2C** depicts speckle contrast images from a series of speckle contrast images and a graph representing a perfusion level based on the series of speckle contrast images. Graph **230** depicts a perfusion measurement of a nailbed, showing a first curve **232** representing the perfusion determined based on uncorrected measurements, and a second curve **234** representing the perfusion determined based on measurements corrected with a motion correction algorithm as described in this disclosure. Around the time mark of 10 seconds, blood flow to the finger is artificially restricted, and about 30 seconds later, the restriction is removed. In particular between the time range of 23 till 38 seconds, the uncorrected perfusion measurement show a number of motion artefacts, where the perfusion seems to sharply rise and fall again.

The figure further shows three exemplary speckle contrast images before (images $236_{1-3}$) and after (images $238_{1-3}$) processing with a motion correction and compensation algorithm based on a single wavelength. In these images, a light colour represents a low speckle contrast, and hence a high perfusion, while a dark colour represents a high speckle contrast, and hence a low perfusion. The three uncorrected images appear more or less the same, making it difficult for a user to recognise a time (or in other applications, a region) with low or high perfusion. By contrast, the motion corrected images display a clear difference between the (middle) image acquired during restriction of the blood flow and the other images with unrestricted blood flow, allowing a user to select a time or place with a high perfusion.

Additionally, anatomical structures such as the edges of the finger and the nailbed can more easily be recognised in the motion compensated image, while details may be hard to recognise in the uncorrected images due to their grainy nature. This further facilitates interpretation of the images by a user.

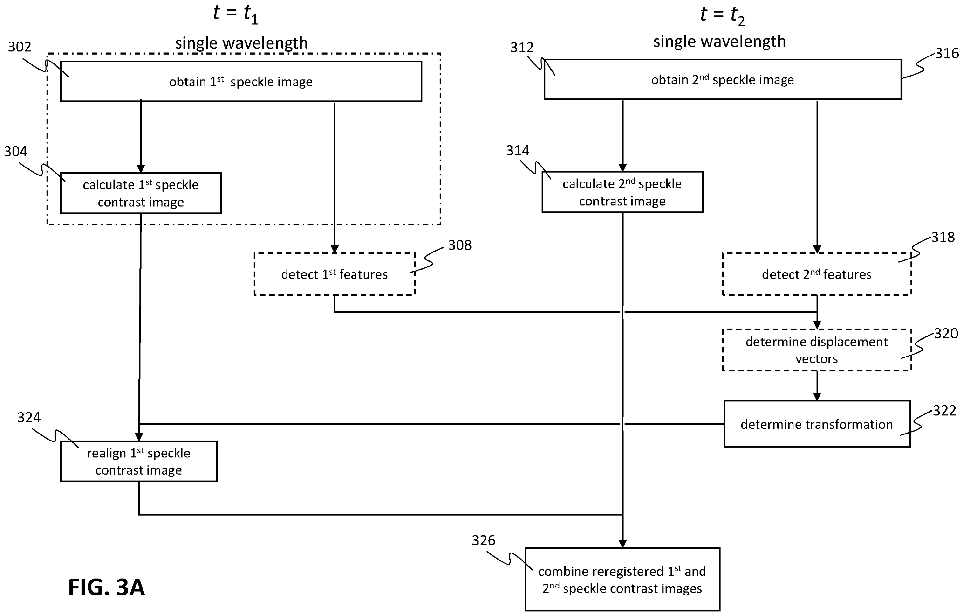

**Fig. 3A** depicts a flow diagram for laser speckle contrast imaging according to an embodiment of the invention. In a first step **302**, a first raw speckle image may be obtained at a first time instance $t = t_1$. A light source may illuminate a target area with coherent light of a predetermined wavelength and an image sensor may capture a first raw speckle image based on the predetermined wavelength. Based on the first raw speckle image, a first laser speckle contrast image may be computed **304**. A laser speckle contrast image may be determined, for example, by determining a relative standard deviation of pixel values in a sliding window, e.g. a $3 \times 3$ window, a $5 \times 5$ window, or a $7 \times 7$ window. Generally, a $(2n + 1) \times (2n + 1)$ window may be selected for a natural number n, depending on the speckle size. Alternatively, a convolution with a kernel may be used, where the size of the kernel may be selected based on the speckle size. A relative standard deviation may be determined by computing the standard deviation of pixel intensity values in an area divided by the mean pixel intensity value in the area. Alternatively, laser speckle contrast values may be determined in any other suitable way.

In a next step **308**, a processor may determine a first plurality of first features in the first raw speckle image. Alternatively, the first plurality of first features may be determined in the first speckle contrast image. Preferably, the image with the most clearly defined anatomical features is used; it may depend on the imaging parameters such as wavelength and exposure time whether the anatomical have a higher visual distinctiveness in the raw speckle image or in the speckle contrast image. In the remaining part of the description of **Fig. 3A**, the term 'speckle image' may refer to either a raw speckle image or a speckle contrast image. The steps relating to feature detection will be described in more detail below with reference to **Fig. 5-6**.

Steps **312-318** are analogous to steps **302-308**, respectively, executed at a second time instance $t = t_2$. Thus, a second raw speckle image may be obtained **312** at the second time instance $t = t_2$. The light source may illuminate the target area with coherent light of the predetermined wavelength and the image sensor may capture a second raw speckle image based on the predetermined wavelength. Based on the second raw speckle image, a second laser speckle contrast image may be computed **314**.

In a next step **318**, the processor may determine a second plurality of second features in the second speckle image. At least a part of the second plurality of second features should correspond to at least a part of the first plurality of first features. Typically, the second speckle image is similar to the first speckle image, as in a typical application, the target area will not change much between $t = t_1$ and $t = t_2$. Therefore, when a deterministic algorithm is used to detect features, most of the features detected in the second speckle image will generally correspond to features detected in the first speckle image, in the sense that the detected features in the images represent the same, or practically the same, anatomical features in the imaged target. Thus, a plurality of second features may be associated with a plurality of first features.

In a next step **320**, the processor may determine a plurality of displacement vectors based on the first features and the corresponding second features, a displacement vector describing the displacement of a feature relative to an image. For example, the processor may determine pairs of features comprising one first feature and one second feature, determine a first position of the first feature relative to the first speckle image, determine a second position of the second feature relative to the second speckle image, and determine a difference between the first and second positions. Typically, pairs of corresponding features may be pairs of a first feature and an associated second feature representing the same anatomical feature.

In a next step **322**, the processor may determine a transformation, e.g. an affine transformation or a more general homography, for registering corresponding features with each other, based on the plurality of displacement vectors. The transformation may e.g. be found by selection from a class of transformations a transformation that minimizes a distance between pairs of corresponding features. Based on the transformation, the processor may register or align **324** the first laser speckle contrast image and the second laser speckle contrast image with each other, by transforming the first and/or second laser speckle contrast images. Typically, the older image may be transformed to be registered with the newer image.

In other embodiments, steps **308** and **318** may be omitted, and displacement vectors may be determined based on the first and second speckle images. For example, a dense optical flow algorithm may be used, such as a Pyramid Lucas-Kanade algorithm or a Farneback algorithm to determine displacement vectors. This sort of algorithms typically performs a convolution of a pixel neighbourhood from the first speckle image with a part or the whole of the second speckle image, thus matching a neighbourhood for each pixel in the first speckle image with a neighbourhood in the second speckle image. Such methods may also comprise determining a polynomial expansion to model pixel values in pixel neighbourhoods in the first and second speckle images, comparing expansion coefficients, and determining displacement vectors based on the comparison.

This way, displacement vectors may be determined for e.g. individual pixels or groups of pixels, based on pixel values in groups of pixels in the speckle images.

In some embodiments, step **320** may be omitted, and a transformation may be determined based on pixel values of groups of pixels in the first speckle image and pixel values of associated groups of pixels in the second speckle image, for instance using a trained neural network that receives a first image and a second image as input and provides as output a transformation to register the first image with the second image or, alternatively, the second image with the first image.

The processor may then compute **326** a combined, e.g. averaged, laser speckle contrast image based on the registered first and second laser speckle contrast images. Computing a combined image may comprise computing a weighted average, the weights preferably being based on a normalised amount of speckle contrast, on a relative change in speckle contrast, on the determined displacement vectors, and/or on parameters associated with the determined transformation. Computing a combined image may further comprise applying one or more filters, e.g. a median filter, or an outlier filter.

In other embodiments, the steps may be performed in a different order. For example, the laser speckle contrast images may be computed after the raw speckle images have been registered. This way, temporal or spatio-temporal speckle contrast images may be computed. However, if the transformation is more general than translation and rotation (e.g. comprises scaling or shearing), the transformation may distort the speckle pattern and thus introduce a source of noise. In some embodiments, a single laser contrast raw speckle image may be computed based on the combined, e.g. averaged, raw speckle images. In this case, the images are preferably registered with sub-pixel accuracy.

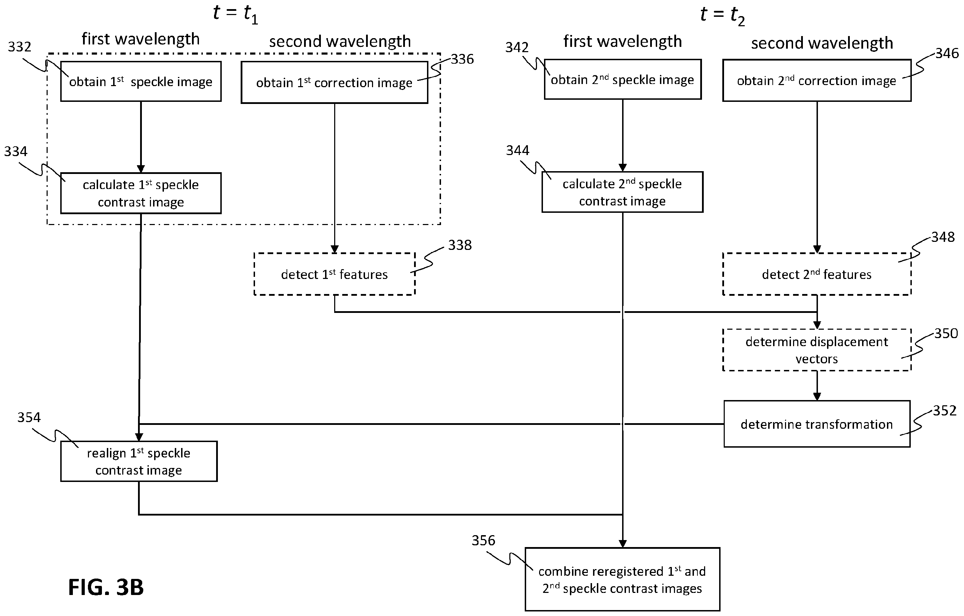

**Fig. 3B** depicts a flow diagram for laser speckle contrast imaging according to an embodiment of the invention. In a first step **332**, a first raw speckle image may be obtained at a first time instance $t = t_1$. A first light source may illuminate a target area with coherent light of a first wavelength and a first image sensor may capture a first raw speckle image based on the first wavelength. Based on the first raw speckle image, a first laser speckle contrast image may be computed **334**. A laser speckle contrast image may be determined as described above with reference to step **304**.

In a next step **336**, a first correction image associated with the first speckle image may be obtained at a first time instance $t = t_1$. The first correction image may comprise pixels with pixel values and pixel coordinates, the pixel coordinates identifying the position of the pixel in the image. The first correction image is preferably obtained simultaneously with the first raw speckle image, but in an alternative embodiment, the first correction image may be obtained e.g. before or after the first raw speckle image.

A second light source may illuminate the target area with light of at least a second wavelength, different from the first wavelength and a second image sensor may capture a first correction image based on the at least second wavelength. The second light source may use coherent light or incoherent light. The second light source may generate monochromatic light or polychromatic light, e.g. white light. The second image sensor may be the same sensor as the first image sensor, or a different sensor.

In the embodiment described above with reference to **Fig. 3A**, the second wavelength is the same as the first wavelength, and the first correction image is the same image as the first raw speckle image.

In a next step **338**, a processor may determine a first plurality of first features in the first correction image. The steps relating to feature detection will be described in more detail with reference to **Fig. 5-6.**

Steps **342-348** are analogous to steps **332-338**, respectively, executed at a second time instance $t = t_2$. Thus, a second raw speckle image may be obtained **342** at the second time instance $t = t_2$. The first light source may illuminate the target area with coherent light of the first wavelength and the first image sensor may capture a second raw speckle image based on the first wavelength. Based on the second raw speckle image, a second laser speckle contrast image may be computed **344.**

In a next step **346**, a second correction image associated with the second raw speckle image may be obtained at the second time instance $t = t_2$. The second light source may illuminate the target area with light of the at least second wavelength and the second image sensor may capture a second correction image based on the at least second wavelength.

In a next step **348**, the processor may determine a second plurality of second features in the second correction image. At least a part of the second plurality of second features should correspond to at least a part of the first plurality of first features. Typically, the second correction image is similar to the first correction image, as in a typical application, the target area will not change much between $t = t_1$ and $t = t_2$. Therefore, when a deterministic algorithm is used to detect features, most of the features detected in the second correction image will generally correspond to features detected in the first correction image, in the sense that the detected features in the images represent the same, or practically the same, anatomical features in the imaged target. Thus, a plurality of second features may be associated with a plurality of first features.

In a next step **350**, the processor may determine a plurality of displacement vectors based on the first features and the corresponding second features, a displacement vector describing the displacement of a feature relative to an image. For example, the processor may determine pairs of features comprising one first feature and one second feature, determine a first position of the first feature relative to the first correction image, determine a second position of the second feature relative to the second correction image, and determine a difference between the first and second positions. Typically, pairs of corresponding features may be pairs of a first feature and an associated second feature representing the same anatomical feature.

In a next step **352**, the processor may determine a transformation, e.g. an affine transformation or a more general homography, for registering corresponding features with each other, based on the plurality of displacement vectors. The transformation may e.g. be found by selection from a class of transformations a transformation that minimizes a distance between pairs of corresponding features. Based on the transformation, the processor may register or align **354** the first laser speckle contrast image and the second laser speckle contrast image with each other, by transforming the first and/or second laser speckle contrast images. Typically, the older image may be transformed to be registered with the newer image. In embodiments with more than one image sensor, the transformation parameters determined based on the correction images may be adjusted to account for differences between the fields of view of the more than one image sensor.

In other embodiments, steps **338** and **348** may be omitted, and displacement vectors may be determined based on the first and second correction images. For example, a dense optical flow algorithm may be used, such as a Pyramid Lucas-Kanade algorithm or a Farneback algorithm to determine displacement vectors. This sort of algorithms typically performs a convolution of a pixel neighbourhood from the first correction image with a part or the whole of the second correction image, thus matching a neighbourhood for each pixel in the first correction image with a neighbourhood in the second correction image. Such methods may also comprise determining a polynomial expansion to model pixel values in pixel neighbourhoods in the first and second correction images, comparing expansion coefficients, and determining displacement vectors based on the comparison.

This way, displacement vectors may be determined for e.g. individual pixels or groups of pixels, based on pixel values in groups of pixels in the first correction image and associated groups of pixels in the second correction image.

In some embodiments, step **350** may be omitted, and a transformation may be determined based on pixel values of groups of pixels in the first correction image and pixel values of associated groups of pixels in the second correction image, for instance using a trained neural network that receives a first image and a second image as input and provides as output a transformation to register the first image with the second image or alternatively, the second image with the first image.

The processor may then compute **356** a combined, e.g. averaged, laser speckle contrast image based on the registered first and second laser speckle contrast images, as described above with reference to step **326.**

In other embodiments, the steps may be performed in a different order. For example, the laser speckle contrast images may be computed after the raw speckle images have been registered. This way, temporal or spatio-temporal speckle contrast images may be computed. However, if the transformation is more general than translation and rotation (e.g. comprises scaling or shearing), the transformation may distort the speckle pattern and thus introduce a source of noise. This is particularly true for embodiments with more than one camera. In some embodiments, a single laser contrast raw speckle image may be computed based on the combined, e.g. averaged, raw speckle images. In this case, the images are preferably registered with sub-pixel accuracy.

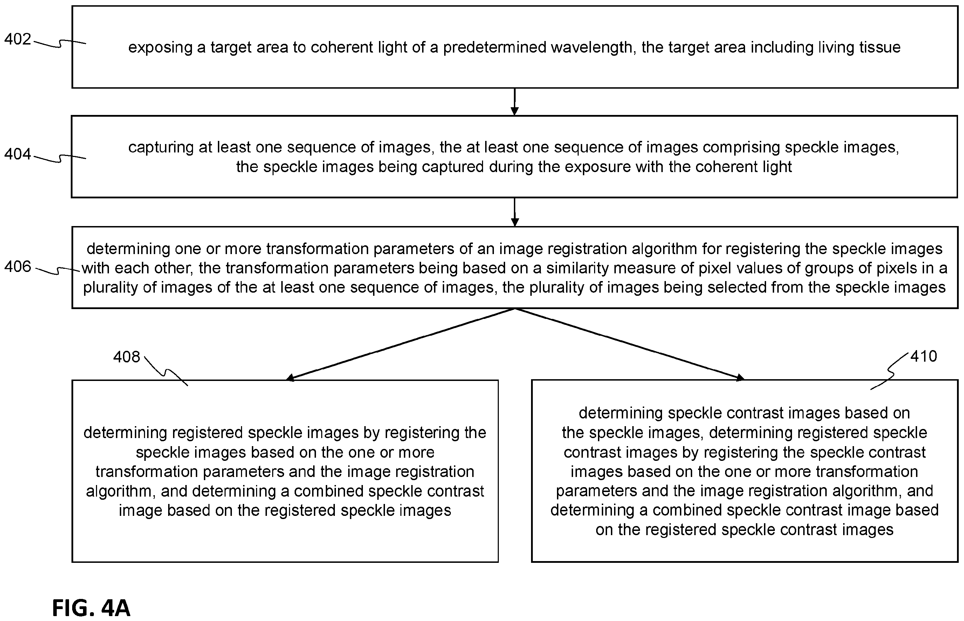

**Fig. 4A** depicts a flow diagram for a motion-compensated speckle contrast imaging method according to an embodiment of the invention. In a first step **402**, the method may comprise exposing a target area to coherent light of a predetermined wavelength, the target area including living tissue. Preferably, the target area comprises living tissue, e.g. skin, burns, or internal organs such as intestines, or brain tissue. Preferably, the living tissue is perfused and/or comprises blood vessels and/or lymph vessels. The predetermined wavelength may be a wavelength in the visible spectrum, e.g. in the red, green, or blue part of the visible spectrum, or the predetermined wavelength may be a wavelength in the infrared part of the spectrum, preferably in the near-infrared part.

In a next step **404**, the method may comprise capturing, e.g. by an image sensor, at least one sequence of images, the at least one sequence of images comprising (raw) speckle images, the (raw) speckle images being captured during the exposure with the first light.

Each raw speckle image may comprise pixels, the pixels being defined by pixel coordinates and having pixel values. The pixel coordinates may define the position of the pixel relative to the image, and are typically associated with a sensor element of the image sensor. The pixel value may represent a light intensity.

The image sensor may comprise a 2D image sensor, e.g. a CCD, for example a monochrome camera or a colour camera. The images in the sequence of images can be frames in a video stream or in a multi-frame snapshot.

In a next step **406**, the method may further comprise determining one or more transformation parameters of an image registration algorithm for registering the speckle images with each other. The transformation parameters may be based on a similarity measure of pixel values of groups of pixels in a plurality of images of the at least one sequence of images, the images in the plurality of images being selected from the speckle images. Alternatively, the images in the plurality of images may comprise other images than the raw speckle images, that are associated with the raw speckle images. The transformation parameters preferably define one or more transformations out the group of homographies, projective transformations, or affine transformations.

The determination of the transformation parameters based on groups of pixels is described in more detail below with reference to step **416**, with the understanding that in this embodiment, the (raw) speckle images are used as the correction images.

The method may further comprise determining a combined laser speckle contrast image based on the at least part of the sequence of first speckle images and the one or more determined transformation parameters, using either step **408** or step **410**.

In a step **408**, the method may further comprise determining registered speckle images by registering the speckle images based on the one or more transformation parameters and the image registration algorithm, and determining a combined speckle contrast image based on the registered speckle images. In such an embodiment, the algorithm may first register the sequence of raw speckle images using the determined transformation, then compute a sequence of speckle contrast images, and then combine the registered speckle contrast images.

In an alternative step **410**, the method may further comprise determining speckle contrast images based on the speckle images, determining registered speckle contrast images by registering the speckle contrast images based on the one or more transformation parameters and the image registration algorithm, and determining a combined speckle contrast image based on the registered speckle contrast images. In other words, the algorithm may first compute a sequence of speckle contrast images, then register the speckle contrast images using the determined transformation, and then combine the registered speckle contrast images.

Further alternatives are discussed below with reference to steps **418** and **420**.

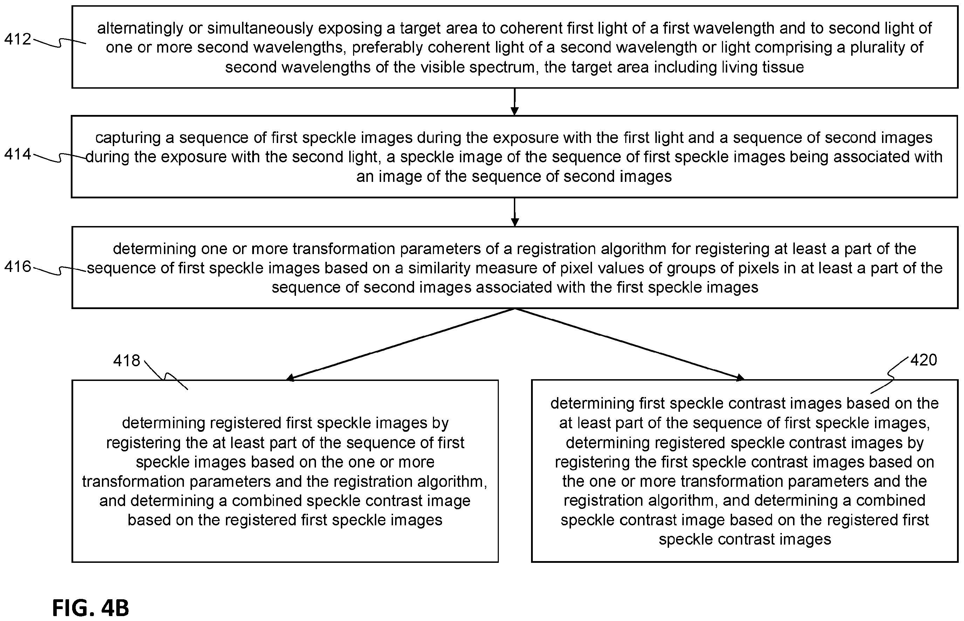

**Fig. 4B** depicts a flow diagram for a motion-compensated speckle contrast imaging method according to an embodiment of the invention. In a first step **412**, the method may comprise alternatingly or simultaneously exposing a target area to coherent first light of a first wavelength and to second light of one or more second wavelengths, preferably at least in part different from the first wavelength. The second light may be, for example, coherent light of a second wavelength, narrow-band light, or light comprising a plurality of second wavelengths of the visible spectrum, e.g. white light. Preferably, the target area comprises living tissue, e.g. skin, burns, or internal organs such as intestines, or brain tissue. Preferably, the living tissue is perfused and/or comprises blood vessels and/or lymph vessels.

In a next step **414**, the method may comprise capturing, e.g. by an image sensor system with one or more image sensors with a fixed relation to each other, a sequence of first (raw) speckle images during the exposure with the first light, and a sequence of second (correction) images during the exposure with the second light. A speckle image of the sequence of first speckle images may be associated with an image of the sequence of second images.

In the case of simultaneous exposure and acquisition, each second image may be associated with the simultaneously acquired first speckle image. In an embodiment where the second images are the same images as the first speckle images, each image may be considered associated with itself, and the image may be referred to as a first speckle image or as a second image, depending on its function in the algorithm (e.g. determining transformation parameters or providing perfusion information).

In the case of alternating acquisition, a first speckle image may be associated with, e.g., the second image acquired immediately preceding or subsequent to the first speckle image, or both. When the first speckle images are acquired at a higher rate than the second images, several first speckle images may be associated with a single second image.

Thus, a sequence of first raw speckle images of the target area and a sequence of correction images of the target area may be acquired, each correction image being associated with one or more first raw speckle images. Each correction image may comprise pixels, the pixels being defined by pixel coordinates and having pixel values. The pixel coordinates may define the position of the pixel relative to the image, and are typically associated with a sensor element of the image sensor. The pixel value may represent a light intensity.

The image sensor system may comprise one or more 2D image sensors, e.g. CCDs. The first raw speckle images and the correction images may be acquired using one or more image sensors, for example using greyscale cameras or colour (RGB) cameras. The images in the sequence of images may be e.g. frames in a video stream or multi-frame snapshot.

In a next step **416**, the method may further comprise determining one or more transformation parameters of a registration algorithm for registering at least a part of the sequence of first speckle images based on a similarity measure of pixel values of groups of pixels in at least a part of the sequence of second images associated with the first speckle images. The transformation parameters preferably define one or more transformations out the group of homographies, projective transformations, or affine transformations.

Determining transformation parameters may comprise selecting a first correction image from the at least part of the sequence of correction images and determining a plurality of first groups of pixels in the first correction image. The first correction image may be a reference correction image. In an embodiment, the first correction image may be the first image, e.g. when a single output image is generated based on input by a user. In a different embodiment, the first correction image may be the most recent correction image, e.g. when a continuous stream of output images is being generated.

A first group of pixels may be associated with a feature in the first correction image, e.g. an edge or corner. Preferably, a feature is associated with a physical or anatomical feature, e.g. a blood vessel, or more in particular, a sharp corner or a bifurcation in a blood vessel. Image features not related to physical features, such as overexposed image parts or edges of speckles, may display a large inter-frame variation, and may hence be less useful to register images. The features may be predetermined features, e.g. features belonging to a class of features, such as corners or regions with large differences in intensity. Features may further be determined by e.g. a quality metric, restrictions on mutual distances between features, et cetera.

Alternatively, a group of pixels may be associated with a region in the first correction image, e.g. a neighbourhood of a predetermined set of pixels, for example, every pixel in the image, or a selection of pixels equally distributed over the image.

Determining transformation parameters may further comprise selecting one or more second correction images, different from the first correction image. For each of the selected one or more second correction images, a plurality of second groups of pixels may be determined. The second groups of pixels may be associated with a feature in the second correction image. If feature-based (image) registration is used, e.g. using a sparse optical flow algorithm, preferably, the same algorithm is used to determine the first groups of pixels and the second groups of pixels.

If the first groups of pixels are determined based on pixel coordinates, the second groups of pixels may be determined by convolving or cross-correlating a first group of pixels with the second correction image and e.g. selecting the group of pixels that is most similar to the first group of pixels, based on a suitable similarity metric. The convolution may be restricted in space, e.g. by only searching for a matching second group of pixels close to the position of the first group of pixels. Alternatively or additionally, the second groups of pixels may be restrained to conserve the mutual orientation of the first groups of pixels, e.g. for preventing anatomically impossible combinations.

The second groups of pixels may then be associated with the first groups of pixels based on, at least, a similarity in pixel values. In an embodiment where the second groups of pixels are determined by matching or convolution with the first groups of pixels, such association may be performed as part of determining the second groups of pixels. If feature-based registration is used, a second group of pixels may be associated with a first group of pixels based on similarity of the feature associated with the second group of pixels and the feature associated with the first group of pixels.

A transformation for registering the second correction image and the first correction image, and hence for registering the associated first speckle images or derived first speckle contrast images, may be determined based on the pixel coordinates of pixels in the associated first and second groups of pixels. Determining a transformation may comprise determining a 3D motion of the image sensor system relative to the target area, or may be informed by the effects this 3D motion would have on the acquired images.

As an intermediate step, displacement vectors may be determined, based on positions of the first and associated second groups of pixels, e.g. based on positions of features in the first and second correction images. The displacement vectors may represent motion of the target area relative to the image sensor or image capturing device. In some embodiments, the neighbourhood of one or more determined object features may be used to determine the displacement vectors and/or the transformation.

The determination of displacement vectors and/or the determination of the transformation may be based on optical flow parameters, determined using any suitable sparse or dense optical flow algorithm. Determining displacement vectors may comprise determining pairs of corresponding or matching features, one feature of a pair of features being determined in a correction image associated with a first time instance, the other feature in the pair of features being determined in the subsequent correction image in the sequence of correction images. associated with a subsequent time instance.

Methods to determine transformation parameters and, optionally, features, are discussed in more detail below with reference to **Fig. 5** and **6.**

The method may further comprise determining a combined laser speckle contrast image based on the at least part of the sequence of first speckle images and the one or more determined transformation parameters, using either step **418** or step **420.**

In a step **418**, the method may further comprise determining registered first speckle images by registering the at least part of the sequence of first speckle images based on the one or more transformation parameters and the registration algorithm, and determining a combined speckle contrast image based on the registered first speckle images. In such an embodiment, the algorithm may first register the sequence of raw speckle images using the determined transformation, then compute a sequence of speckle contrast images, and then combine the registered speckle contrast images.

In an alternative step **420**, the method may further comprise determining first speckle contrast images based on the at least part of the sequence of first speckle images, determining registered speckle contrast images by registering the first speckle contrast images based on the one or more transformation parameters and the registration algorithm, and determining a combined speckle contrast image based on the registered first speckle contrast images. In other words, the algorithm may first compute a sequence of speckle contrast images, then register the speckle contrast images using the determined transformation, and then combine the registered speckle contrast images.

In a further alternative, determining a combined laser speckle contrast image may comprise determining a sequence of registered first raw speckle images based on the first raw speckle images and the determined transformation, determining a combined speckle contrast image based on two or more registered first raw speckle images of the sequence of registered first raw speckle images, and determining a combined speckle contrast image based on the combined raw speckle image. In such an embodiment, the algorithm may first register the sequence of raw speckle images using the determined transformation, then combine the registered speckle contrast images, and then compute a speckle contrast image.

In an even further alternative, the combining and the computing of a speckle contrast may be a single step, e.g. by computing a temporal or spatio-temporal speckle contrast based on the sequence of registered first raw speckle images.

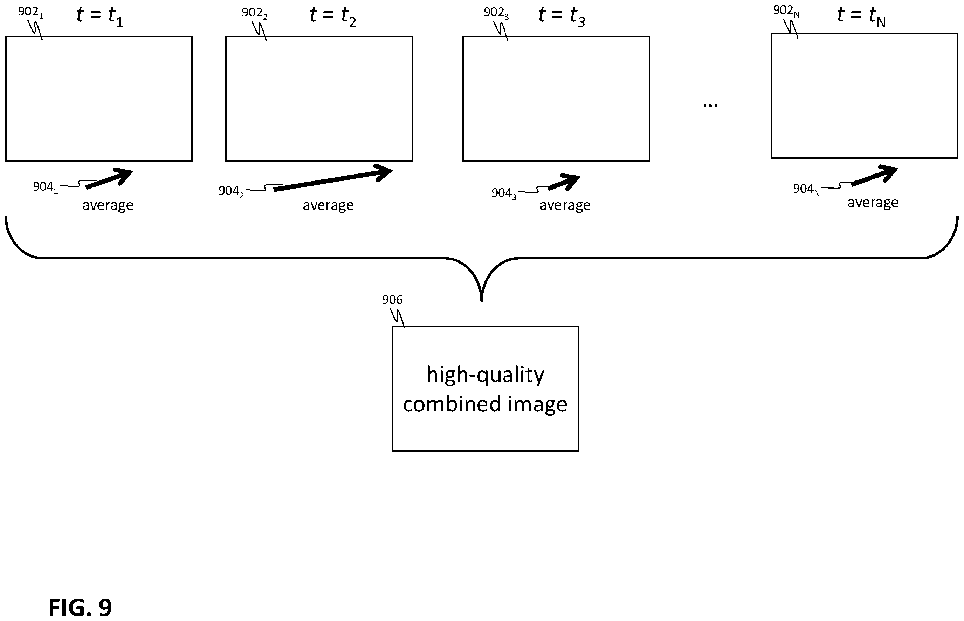

Combining raw speckle images or speckle contrast images may comprise averaging, weighted averaging, filtering with e.g. a median filter, et cetera. Weights for weighted averaging may be based e.g. on a quantity derived from the speckle contrast, derived from the transformation parameters, or derived from the displacement vectors. Methods of combining raw speckle images or speckle contrast images are discussed in more detail below with reference to **Fig. 9.**

**Fig. 5** depicts a method for determining a transformation according to an embodiment of the invention. A first plurality of first features $506_{1-3}$ may be determined in a first correction image $502$, acquired at a first time instance $t = t_1$. Each of the plurality of first features may be associated with a group of pixels in the first correction image. Preferably, the first features relate to anatomical structures, e.g. blood vessels $504_{1-2}$, or other stable features that may be assumed not to move between subsequent frames. Therefore, the first correction image is preferably obtained using light which makes such anatomical structures clearly visible. For example, green light may be used, which is strongly absorbed by blood vessels, but not by most other tissues. Therefore, blood vessels may appear as dark structures in a light environment, resulting in a high visual distinctiveness. However, other embodiments may use light of one or more other wavelengths, e.g. blue light or white light.

Features $506_{1-3}$ may be determined using any suitable feature detection algorithm, for example a feature detector based on a Harris detector or Shi-Tomasi detector, such as goodFeaturesToTrack from the OpenCV library. Other examples of suitable feature detectors and descriptors include Speeded-Up Robust Features (SURF), Features from Accelerated Segment Test (FAST), Binary Robust Independent Elementary Features (BRIEF), and combinations thereof such as ORB. Various suitable algorithms have been implemented in generally available image processing libraries such as OpenCV. Preferably, depending on the application, the algorithm should be fast enough to allow real-time image processing.

Typically, sharp corners (e.g. features $506_{1,3}$) and bifurcations (e.g. feature $506_2$) are good features. Preferably, a deterministic feature detection algorithm is used, i.e., a feature detection algorithm that detects identical features in identical images. Preferably, the features should be distributed over a large part of the image area. A good distribution of feature points over the image may be obtained by requiring a minimum distance between selected feature points. In some embodiments, e.g. based on implementations of BRIEF or ORB, the features may be assigned a descriptor identifying feature properties, facilitating feature distinction and feature matching.

A minimum number of feature points depends on the type of transformation; for example an affine transformation has six degrees of freedom, while a homography has eight degrees of freedom. Thus, the first plurality of first features may comprise at least 5 features, preferably at least 25, more preferably at least 250, even more preferably at least 1000. The number of features may depend on the amount of pixels in the image, with a larger number of features being used for images with more pixels. Typically, a higher number of features may result in a more accurate transformation, as random errors may be averaged out.

However, there are various reasons why the number of features may be limited. For example, there may only be a limited number of features that satisfy predetermined quality indicators, e.g. a magnitude of a local contrast or the sharpness of a corner. Additionally, the computation time increases with the number of features, and hence, the number of features may be limited to allow real-time image registration, e.g. for a 50 fps video feed, the entire algorithm should preferably take less than 20 ms per frame.

A second plurality of second features $516_{1-3}$ associated with second groups of pixels may be determined in a second correction image $512$, acquired at a second time instance $t = t_2$. Preferably, the field of view of the second correction image overlaps substantially, preferably more than half, with the field of view of the first correction image. Preferably, the second features relate to the same anatomical structures, e.g. blood vessels $514_{1-2}$ as the first features. Preferably, the same feature detection algorithm is used to detect features in both the first and second correction images.

The determined first and second features may comprise position information relative to the first and second correction images, respectively. Based on the first plurality of first features $506_{1-3}$ and the second plurality of second features $516_{1-3}$, a plurality of displacement vectors $524_{1-2}$ may be determined, a displacement vector describing the displacement of a feature relative to an image. In an intermediate step, pairs of corresponding features may be determined, e.g. feature $506_1$ may be associated with feature $516_1$, feature $506_2$ may be associated with feature $516_2$, and feature $506_3$ may be associated with feature $516_3$. Pairs of corresponding features may e.g. be determined based on feature parameters such as local contrast or the sharpness of a corner, or based on distances between features in the first and second correction images. In some embodiments, not all features in the first correction image can be paired to features in the second correction image. In some embodiments, determining displacement vectors $524_{1-3}$ and determining pairs of corresponding features may be performed in a single step.

For example, if a minimum distance between features is imposed and the displacement is assumed to be smaller than the minimum distance, an algorithm that minimizes the distance between point clouds formed by, respectively, the first and second features, may implicitly determine pairs of corresponding features and displacement vectors for each pair of corresponding features. In a typical embodiment, the typical inter-frame displacement is only a few pixels. In an embodiment, the plurality of displacement vectors may be filtered to exclude potential outliers, e.g. displacement vectors that deviate more than a predetermined amount from displacement vectors originating from nearby features.

In other embodiments, displacement vectors may be determined based on associated groups of pixels, based on pixel values in the first and second correction images. As was explained above with reference to **Fig. 3**, in such embodiments feature detection may be omitted. Instead, displacement vectors may be determined using e.g. a dense optical flow algorithm, such as a Pyramid Lucas-Kanade algorithm or a Farneback algorithm. In principle, any method to determine displacement vectors based on pixel values of corresponding groups of pixels may be used.

Based on the plurality of displacement vectors $524_{1-3}$, a transformation may be determined. In an embodiment, the transformation may be defined by an average or median displacement vector, or by another displacement vector that is statistically representative of the plurality of displacement vectors. In a different embodiment, the transformation may be an affine transformation, a projective transformation, or a homography, combining e.g. translation, rotation, scaling and shearing transformations. Preferably, the transformation images features from the first correction image onto the corresponding features in the second correction image. The transformation may then be applied to the first raw speckle image to register the first raw speckle image with the second raw speckle image.

In an embodiment, the first and second correction images may be preprocessed before determining features. For example, overexposed and/or underexposed regions may be identified based on pixel values. Subsequently, these regions may be masked, so no features may be detected in those regions. The mask may be slightly larger than the overexposed or underexposed region, e.g. by growing the identified region with a predetermined number of pixels. Masking overexposed and/or underexposed regions may improve the quality of the features, as it prevents features e.g. associated with an edge or corner of an overexposed region.

**Fig. 6A** displays an example of determining a transformation according to an embodiment of the invention, where the determined transformation is a translation. As was explained with reference to **Fig. 5**, a first plurality of first features $602_{1-3}$ may be determined in a first correction image acquired at $t = t_1$, and a second plurality of second features $604_{1-3}$ may be determined in a second correction image acquired at $t = t_2$. Based on corresponding pairs of features, a plurality of displacement vectors $606_{1-3}$ may be determined. For the sake of clarity, only the features and the displacement vectors are shown, and not the (anatomical) structures.

In a typical situation, the determined displacement vectors $606_{1-3}$ will not all be exactly the same. In the depicted example, displacement vector $606_1$ is slightly shorter than average, while displacement vector $606_3$ is slightly larger than average. Similarly, the directions of the displacement vector display some variation. Based on the displacement vectors, an average displacement vector **608** may be determined. A translation may be defined by a single vector. For example, all pixels of the first raw speckle image acquired at $t = t_1$ may be shifted with an amount equal to the average displacement vector. In principle, a translation may be determined based on a single displacement vector. However, by determining a plurality of displacement vectors, the accuracy of the transformation may be improved.

In an embodiment, a similarity between the average displacement vector **608** and the determined displacement vectors $606_{1-3}$ may be computed, for example based on the variation of the displacement vectors. This way, an indication may be obtained how well the transformation compensates for the detected displacement of individual pairs of features. Alternatively, the average distance between the features in the first correction image after transformation and the corresponding features in the second correction image may be determined.

**Fig. 6B** displays an example of determining a transformation according to an embodiment of the invention, where the determined transformation is an affine transformation. Similar to **Fig. 6A**, a first plurality of first features $612_{1-3}$, a second plurality of second features $614_{1-3}$, and a plurality of displacement vectors $616_{1-3}$ may be determined. In this example, however, the average displacement vector **618**, which has almost zero length, is not representative for the determined displacement vectors, which are typically longer, and point in different directions.

Hence, to compensate for this kind of motion, a more general transformation is needed, for example an affine transformation. Affine transformations include translations, rotations, mirroring, scaling, and shearing transformations, and combinations thereof. It is possible to selectively exclude transformations by restricting transformation parameter values. For example, mirroring may be excluded as a possible transformation, as mirroring is typically not physically possible.

In general, an affine transformation can be computed using a transformation matrix with six degrees of freedom as described in equation (1), acting on a point represented in homogeneous coordinates. By restricting the potential values of the affine transformation matrix, the affine transformation may be limited to only predefined operations. For example, a more specific transformation matrix limited tot e.g. rotation matrices can be obtained which can be more suitable for certain applications.

$$\begin{bmatrix} x' \\ y' \\ 1 \end{bmatrix} = \begin{bmatrix} a_{11} & a_{12} & t_x \\ a_{21} & a_{22} & t_y \\ 0 & 0 & 1 \end{bmatrix} \begin{bmatrix} x \\ y \\ 1 \end{bmatrix} \leftrightarrow p' = A\,p \qquad (1)$$

Here, $A$ is a transformation matrix transforming a point $p$ with coordinates $x$ and $y$, typically in pixel coordinates, into a transformed point $p'$ with coordinates $x'$ and $y'$. Matrix $A$ comprises six free parameters, of which $t_x$ and $t_y$ define a translation, while the submatrix $\begin{bmatrix} a_{11} & a_{12} \\ a_{21} & a_{22} \end{bmatrix}$ may define reflections, rotations, scaling and/or shearing. In this case, a transformation size may e.g. be based on a norm of the transformation matrix $A$, or the norm of the matrix $A - I$, where $I$ is the Identity matrix.

To solve equation (1), at least three displacement vectors may be used to provide a solvable system of six equations and six unknowns. Such a linear system can be solved in a deterministic way as is known in the art. In a typical embodiment, many displacement vectors may be determined, each of which may comprise a small error. Therefore, a more robust approach can be to use multiple displacement vectors and use an appropriate fitting algorithm, e.g., least squares fitting as shown in equation (2).

$$[A] = \arg(\min_A(\textstyle\sum_i \lVert p_i' - A\,p_i \rVert^2)) \qquad (2)$$

The reliability of the determined transformation may again be determined as was explained above with reference to **Fig. 6A.**

**Fig. 6C** displays an example of determining a transformation according to an embodiment of the invention, where the determined transformation is a projective transformation. Projective transformations include and are more general than affine transformations. Projective transformation include e.g. skewing transformations. They may be needed to compensate for e.g. a change in angle between the camera and the target area.

Similar to **Fig. 6A** and **6B**, a first plurality of first features **622₁₋₃**, a second plurality of second features **624₁₋₃**, and a plurality of displacement vectors **626₁₋₃** may be determined. In this example, the translation on the left side of the image is much smaller than on the right side of the image. Thus, applying an average translation would transform pixels on the left too much, and pixels on the right not enough. This kind of displacement may be corrected by a projective transformation. Various methods to determine a projective transformation based on four or more displacement vectors are known in the art.

In general, a projective transformation can be calculated using a projective matrix as depicted in equation (3) using inhomogeneous coordinates $(x_1, y_1, z_1)$ and $(x_2, y_2, z_2)$.

$$\begin{bmatrix} x_2 \\ y_2 \\ z_2 \end{bmatrix} = \begin{bmatrix} h_{11} & h_{12} & h_{13} \\ h_{21} & h_{22} & h_{23} \\ h_{31} & h_{32} & h_{33} \end{bmatrix} \begin{bmatrix} x_1 \\ y_1 \\ z_1 \end{bmatrix} \qquad (3)$$

The inhomogeneous coordinates $(x_1, y_1, z_1)$ may be related to pixel coordinates $(x, y)$ of a feature via $x_1 = x$, $y_1 = y$, and $z_1 = 1$, while the transformed pixel coordinates $(x', y')$ may be obtained from the inhomogeneous coordinates via $x' = x_2/z_2$, and $y' = y_2/z_2$. In some embodiments, displacement vectors may be determined by $(x' - x, y' - y)$. In other embodiments, displacement vectors are not explicitly constructed.

Thus, equation (3) may be rewritten as two independent equations as is shown in equation (4).

$$x'(h_{31}x + h_{32}y + h_{33}) = h_{11}x + h_{12}y + h_{13} \qquad (4)$$

$$y'(h_{31}x + h_{32}y + h_{33}) = h_{21}x + h_{22}y + h_{23}$$

Solving for H, equation (4) can be rewritten as equation (5):

$$v_x \boldsymbol{h} = 0 \qquad\qquad (5)$$
$$v_y \boldsymbol{h} = 0$$

where

$$\boldsymbol{h} = (h_{11}, h_{12}, h_{13}, h_{21}, h_{22}, h_{23}, h_{31}, h_{32}, h_{33})^{\mathrm{T}}$$
$$\boldsymbol{v_x} = (-x, -y, -1, 0, 0, 0, x'x, x'y, x') \qquad\qquad (6)$$
$$\boldsymbol{v_y} = (0, 0, 0, -x, -y, -1, x', y, x')$$

Using a set of N feature points, a system of equations can be setup as shown in equation (7):

$$V\boldsymbol{h} = 0 \qquad\qquad (7)$$

where

$$V = \left(\boldsymbol{v_{x1}}, \boldsymbol{v_{y1}}, \boldsymbol{v_{x2}}, \boldsymbol{v_{y2}}, \dots, \boldsymbol{v_{xN}}, \boldsymbol{v_{yN}}\right)^{\mathrm{T}} \qquad\qquad (8)$$

Since by definition the matrix H is homogeneous and can be scaled by any constant, the projective transformation matrix contains eight degrees of freedom. Thus, equation (7) may be solved using only four sets of coordinates provided by four features. The projective transformation matrix H may be normalized using, for example, equation (9):

$$h_{33} = 1 \qquad\qquad (9)$$

or equation (10):

$$h_{11}{}^2 + h_{12}{}^2 + h_{13}{}^2 + h_{21}{}^2 + h_{22}{}^2 + h_{23}{}^2 + h_{31}{}^2 + h_{32}{}^2 + h_{33}{}^2 = 0 \qquad\qquad (10)$$

Equation (7) can be solved deterministically using at least four points, but using more points may result in a more robust result, similar to what was explained above with regards to the affine transformation. In the case of projective transformations or homographies, this may be done using singular value decomposition (SVD). In some embodiments, the step of determining the displacement vectors or point pairs is combined with the determination of the homography step to find the most accurate projective transformation, this can be done with algorithms such as RANSAC.

In an embodiment, an algorithm may first compute a relatively simple transformation, e.g. a translation. The algorithm may then determine whether the computed transformation reproduces the determined displacement vectors with sufficient accuracy. If not, the algorithm may attempt a more general transformation, e.g. an affine transformation, and repeat the same procedure. This may reduce the required computation time if translations are sufficient in a large enough number of cases. In a different embodiment, the algorithm may always compute a general transformation, e.g. always a general homography. This may result in more accurate registration of the raw speckle images or speckle contrast images.

**Fig. 6D** displays an example of determining a plurality of transformations according to an embodiment of the invention. Each correction image **650** in the series of correction images may be divided into a plurality of regions **652$_{1\text{-}n}$**, preferably a plurality of disjoint regions which jointly cover the entire image, for example a rectangular grid. Subsequently, a transformation **654$_1$**, **654$_2$**, ..., **654$_n$** may be determined for each region **654$_1$**, **654$_2$**, ..., **654$_n$**, respectively, preferably in the same manner as was explained above with reference to Fig. 6A-C for the entire image. Subsequently, each region may be transformed using the transformation determined for that region. Alternatively, the determined transformations may be assigned to e.g. a central pixel in the region, and the remaining pixels are transformed according to an interpolation scheme, based on the transformation of the region comprising the pixel and the transformations of neighbouring regions.

In an embodiment, each region may be a single pixel. In such an embodiment, the transformation may be determined based on the pixel value and based on pixel values of pixels in a region surrounding the pixel.

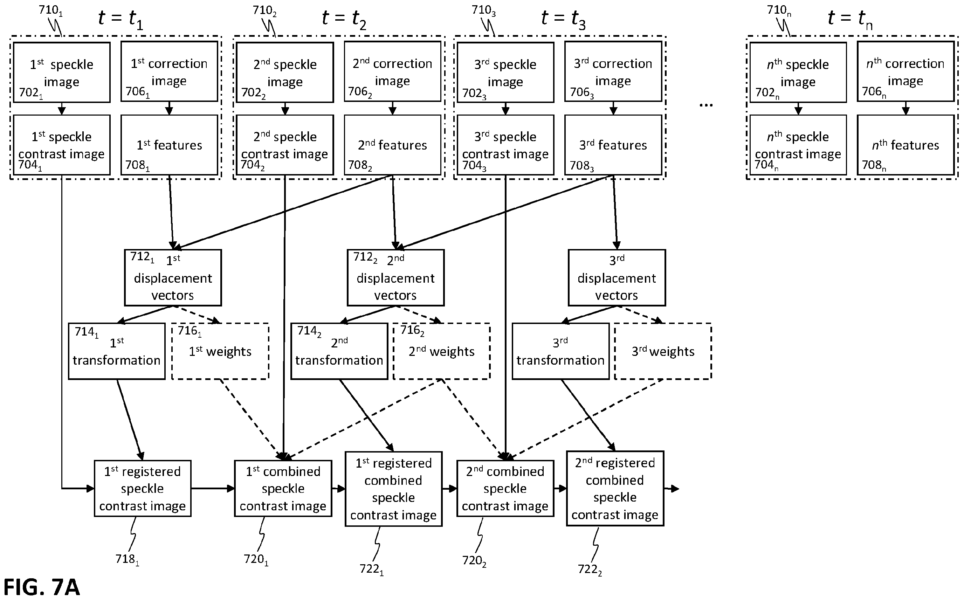

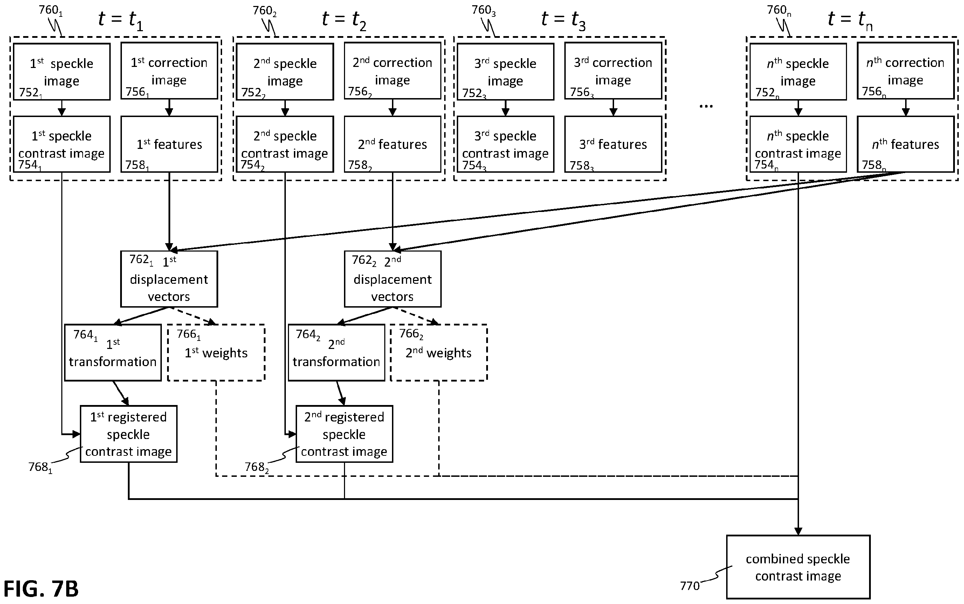

**Fig. 7A** and **7B** depict flow diagrams for laser speckle contrast imaging combining more than two raw speckle images according to an embodiment of the invention. At a first time instance $t = t_1$, a first raw speckle image $702_1$ based on light of a first wavelength may be obtained, and may be used to compute a first laser speckle contrast image $704_1$. A first correction image $706_1$ based on light of at least a second wavelength, and associated with the first raw speckle image may be obtained and a first plurality of first features may be detected $708_1$ in the first correction image. Items $702_1$-$708_1$ may be acquired by performing step $710_1$, which may be similar to steps **302-308** as described with reference to **Fig. 3.** In some embodiments, the first correction image and the first raw speckle image may be the same image.

At a second time instance $t = t_2$, step $710_1$ may be repeated as step $710_2$, resulting in a second raw speckle image $702_2$ based on light of the first wavelength, a second laser speckle contrast image $704_2$, a second correction image $706_2$ based on light of the at least second wavelength, and a plurality of second features $708_2$. Based on the plurality of first features $708_1$ and the plurality of second features $708_2$, a plurality of first displacement vectors $712_1$ may be computed. Based on the plurality of first displacement vectors, a first transformation $714_1$ may be determined, which may be used to transform the first laser speckle contrast image $704_1$ to register the first laser speckle contrast image with the second laser speckle contrast image $704_2$, resulting in a first registered laser speckle contrast image $718_1$. As was discussed above, in some embodiments, a transformation may be determined without explicitly detecting features and/or displacement vectors.

Optionally, first weights $716_1$ may be determined based on the plurality of first displacement vectors $712_1$. A weight may be correlated, preferably inversely correlated, to a length of a representative displacement vector, e.g., a maximum, an average or a median displacement vector, or to e.g. an average or median length of the plurality of first displacement vectors. In an embodiment where the image is divided into a plurality of regions and a transformation is determined for each region, as explained above with reference to **Fig. 6D**, a weight may be determined for each region based on transformation parameters associated with that region or a single weight may be determined for the entire image, e.g. based on a representative parameter, e.g. the largest, average, or median displacement. Determination of a weighted average is discussed in more detail below with reference to **Fig. 9.**

The first registered laser speckle contrast image $718_1$ may then be combined with the second laser speckle contrast image $704_2$, resulting in a first combined laser speckle contrast image $720_1$. The combined laser speckle contrast image can be, e.g., a pixel-wise average or maximum of the first registered laser speckle contrast image $718_1$ and the second laser speckle contrast image $704_2$. Optionally, first weights $716_1$ and/or second weights $716_2$ may be used to determine a weighted average.

At a third time instance $t = t_3$, step $710_1$ may be repeated as step $710_3$, resulting in a third raw speckle image $702_3$ based on light of the first wavelength, a third laser speckle contrast image $704_3$, a third correction image $706_3$ based on light of the at least second wavelength, and a plurality of second features $708_3$. Based on the plurality of second features $708_2$ and the plurality of third features $708_3$, a plurality of second displacement vectors $712_2$ may be computed. Based on the plurality of second displacement vectors, a second transformation $714_2$ may be determined. The second transformation may be used to transform the first combined laser speckle contrast image $720_1$ to register the first combined laser speckle contrast image with the third laser speckle contrast image $704_3$, resulting in a first registered combined laser speckle contrast image $722_1$. The first registered laser speckle contrast image $718_1$ may then be combined with the second laser speckle contrast image $704_2$, resulting in a first combined laser speckle contrast image $720_1$.

The first registered combined laser speckle contrast image $720_1$ may then be combined with the third laser speckle contrast image $704_3$, resulting in a second combined laser speckle contrast image $720_2$. Thus, the second combined laser speckle contrast image may comprise information from the first laser speckle contrast image $704_1$, the second laser speckle contrast image $704_2$, and the third laser speckle contrast image $704_3$. By repeating these steps, the $n^{th}$ image may comprise information from all previous $n-1$ images. Preferably, the weighing may then be skewed to give recent images a higher weight then older images. This embodiment is particularly useful for streaming video, where each captured frame is processed and outputted with a minimal delay. Another advantage of this method is that between two subsequent frames, motion may be assumed to be relatively small, which may speed up processing, and which may allow a wider range of algorithms to be used, as some algorithms may work less well for large motions. In general, feature-based algorithms may be more reliable for relatively large displacements.

**Fig. 7B** depicts a flow diagram for an alternative method for laser speckle contrast imaging combining more than two raw speckle images according to an embodiment of the invention. In this embodiment, a predetermined number of images is combined into a single combined image. Steps $752_{1\text{-}n}$-$760_{1\text{-}n}$ relating to image acquisition, computation of speckle contrast images, and determination of features, may be the same as steps $702_{1\text{-}n}$-$710_{1\text{-}n}$, explained above with reference to **Fig. 7A.**

However, different from the method depicted in **Fig. 7A**, all displacement vectors $762_{1,2}$ are determined relative to a single reference image, e.g. the first or last image in the sequence of $n$ images. In the depicted example, the images are registered with the $n^{th}$ image. Thus, by applying transformations $764_{1,2}$ to the first and second speckle contrast images, respectively, the first and second speckle contrast images are registered with the $n^{th}$ speckle contrast image. Consequently, the weights $766_{1,2}$ are also determined based on a transformation parameter, e.g. average displacement, relative to the $n^{th}$ image. In a final step, all $n$ registered speckle contrast images may be combined into a single combined speckle contrast image **770**.

The method depicted in **Fig. 7B** may result in a combined image having a higher image quality than the method depicted in **Fig. 7A**, but at the cost of a larger delay between image capture and display. Thus, this method is especially advantageous for recording snap shots.

In an embodiment, the method depicted in **Fig. 7B** may be applied on a sliding group of images, e.g. the last $n$ images of a video feed. To keep the time delay low, in this case, $n$ should preferably be not too large, e.g. $n$ may be about 5-20 when the frame rate is e.g. 50-60 fps. Of course, the number of frames which may be processed also depends on the hardware and the algorithm, so larger amounts of frames may still be feasible. Thus, some of the advantages of both methods may be combined. An advantage of using a relatively small number of frames is that dynamic phenomena, e.g. the effect of a heartbeat, may be imaged. An advantage of a larger amount of frames, is that such transient effects may be filtered out, especially when the number of frames is selected to cover an integer multiple of heartbeats and/or respiration cycles.

**Fig. 8** depicts a flow diagram for computing a corrected laser speckle contrast image according to an embodiment of the invention. In general, one may be interested in the relative motion of one or more objects in the target area relative to one or more other objects in the target area; for example, motion of a bodily fluid or red blood cells relative to a tissue. In such a case, noise in a signal derived from the moving object (desired signal) may be compensated by a signal derived from a reference object (reference signal). The underlying principle is that the desired signal may comprise a first component based on the motion of the quantity of interest relative to the reference object, and a second component based on the motion of the entire target area relative to the camera. The reference signal may comprise only, or mainly, a component based on the motion of the entire target area relative to the camera. The reference signal may therefore be correlated to the second component of the desired signal. This correlation may be used to correct or compensate the desired signal. For example, a correction term based on the signal strength of the reference signal may be added to the desired signal.

In the embodiment depicted in **Fig. 8**, a target area is illuminated with coherent light of a first wavelength **802**, e.g. red or infrared light, and illuminated with coherent light of a second wavelength **812**, e.g. green or blue light. Preferably, the light of the first wavelength is mostly scattered by the object or fluid of interest, e.g. blood. Preferably, the light of the second wavelength is mostly scattered by the surface of the target area and/or mostly absorbed by the object or fluid of interest. Preferably, the second wavelength is selected such that the reflection of the second wavelength by blood is at least 25%, at least 50%, or at least 75% less than the reflection by tissue. Preferably, the target area is illuminated with light of the first and second wavelengths simultaneously.

The scattered light of the first wavelength may result in a first raw speckle image, which may be captured **804** by a first image sensor. The scattered light of the second raw speckle image may be captured **814** by a second image sensor, preferably different from the first image sensor. The first raw speckle image may be referred to as a desired signal raw speckle image, while the second raw speckle image may be referred to as a reference signal image or a correction signal image.

Based on the first raw speckle image, a first speckle contrast image may be calculated **806**. Based on the second raw speckle image, a second speckle contrast image may be calculated **806**. Preferably, the speckle contrast is calculated in the same way for the first and second raw speckle images. Speckle contrast may be calculated, for example, in the way that has been explained above with reference to **Fig. 1** and step **304** of **Fig. 3A.**

In a next step **808**, a corrected speckle contrast image may be calculated based on the first and second speckle contrast images. Calculating a corrected speckle contrast image may comprise e.g. adding a correction term or multiplying by a correction factor. A correction term or correction term may be based on a determined amount of speckle contrast in the speckle contrast image in comparison with a reference amount of speckle contrast. The reference amount of speckle contrast may e.g. be predetermined, or may be determined dynamically based on e.g. the amount of speckle contrast in a number of preceding second contrast images, or based on an speckle contrast image with very little motion as determined by e.g. the motion correction algorithm as has been described above.

The corrected speckle contrast image may then be stored **810** for further processing, e.g. reregistration or realignment and temporal averaging as was explained with reference to **Fig. 3A** and **B**. The second raw speckle image and/or the second speckle contrast image may also be stored **818** for further processing, e.g. to determine displacement vectors in a plurality of second raw speckle images to reregister or realign a plurality of simultaneously captured corrected speckle contrast images. In an embodiment, steps **802-818** may replace steps **332-336** in **Fig. 3B.**

Thus, it is an advantage of the methods in this disclosure that the second wavelength image may be used both for multi-spectral coherent correction (or 'dual laser correction'), as explained with reference to **Fig. 8**, and for registering speckle contrast images as explained with reference to **Fig. 3A** and **B**.

**Fig. 9** schematically depicts determining motion compensate speckle contrast image based on a weighted average, according to an embodiment of the invention. For each correction image $902_{1-N}$, a transformation size may be determined based on parameters defining the determined transformation, and/or on the plurality of displacement vectors.

For example, the transformation size may be based on the lengths of the plurality of displacement vectors or a statistical representation, e.g. an average $904_{1-N}$ thereof, or on a matrix norm of a matrix representing the transformation.

The combined speckle contrast image 906 may be a weighted average of the speckle contrast images in the sequence of registered speckle contrast images, each image being weighted with a weight parameter $w'$.

The weight parameter $w'$ may be determined based on the displacement vectors $\|p_i' - p_i\|$, with a high displacement corresponding to a small weight and vice versa, e.g. as defined in equation (11):

$$w' = \frac{1}{P} \sum_{i=1}^{P} \frac{1}{\|p_i' - p_i\|} \qquad (11)$$

Here, the displacement vectors may be defined by a total of $P$ points $p_i'$ which are defined by the coordinates $(x_i', y_i')$ on a reference image and points $p_i$ which are defined by the coordinates $(x_i, y_i)$ on the image that is to be transformed to be registered to the reference image.

The weight parameter $w'$ may also be determined based on a dense or sparse optical flow parameter defining the optical flow between a first image, typically a reference image, and a second image. For example, a weight may be inversely correlated to the average optical flow over all pixels in the image or in a region of interest in the image, e.g. as defined in equation (12):

$$w' = \frac{1}{W \cdot H} \sum_{i,j} \frac{1}{\|v_{ij}'\|} \qquad (12)$$

Here, $v_{ij}'$ is the optical flow of a pixel $(i, j)$ comprising an $x$ and an $y$ component of the optical flow, and $W$ and $H$ are respectively the width and height in pixels of the reference image or the reference region of interest.

Alternatively, a weight parameter may be determined for each pixel, e.g., based on the optical flow per pixel as defined in equation (13):

$$w_{ij}' = \frac{1}{\|v_{ij}\|} \qquad (13)$$

Instead of determining a weight parameter for each pixel or for the image as a while, a weight parameter may also be determined for predefined regions of the image such as a rectangular or triangular mesh. For instance, a weight parameter may be based on the average optical flow in a corresponding predefined region.

The weight parameter may also be determined based on the speckle contrast values $s_{ij}'$, preferably the weight parameter being proportional to the speckle contrast magnitude, e.g. as defined in equation (14):

$$w' = \frac{1}{W \cdot H} \sum_{i,j} \|s_{ij}'\| \qquad (14)$$

Here, $s_{ij}'$ is the speckle contrast of the reference image and $W$ and $H$ are respectively the width and height in pixels of the reference image or the reference region of interest. An advantage of using the speckle contrast is that any noise occurring in a small temporal window on the speckle contrast would blur the speckle contrast. Such noise could be due to motion of e.g. the images object or the camera, or to other sources such as loose fibre connections. An advantage of using weights based on displacement vectors or optical flow is that they may have a higher temporal resolution for LSCI, while a speckle contrast-based weight may lag behind, especially when the perfusion is increasing.

Alternatively, the weight parameter can be determined based on a speckle contrast per pixel.

$$w_{ij}' = \left\| \frac{1}{s_{ij}} \right\| \qquad (15)$$

Alternatively, the weight parameter can be determined based on the average speckle contrast in predefined regions such as a square grid or triangular mesh. Weight parameters may also be determined for dynamically determined regions, where regions may e.g. be determined based on the detected motion.

In an embodiment, various of these weights may be combined. For example, the weights could be normalised and added, multiplied, or compared, selecting e.g. the lowest weight. Alternatively, a first weight, e.g. based on speckle contrast values, may be used to filter out images that do not meet a predefined quality standard, e.g. images having a reduction in contrast magnitude exceeding a predetermined threshold receiving a weight of 0 and all other images receiving a weight of 1. Subsequently, an optical flow or displacement based weight may be used to determine a weighted average of the images that have not been filtered out.

A weighted average may be determined by using a single weight factor per image using a buffer of $N$ images $Img_k$ and a buffer of corresponding $N$ weight factors $w_k$, with $k = 1, 2..., N$. For every new image that is acquired, the following steps may be performed:

1) adding the new image to the buffer as $Img_{N+1}$, which may be selected as a reference image;
2) removing a first weight factor $w_1$ and a first image $Img_1$ from the buffer;
3) applying a geometrical transformation to the other images $Img_2$, $Img_3$, ..., $Img_N$ in the buffer to register them to reference image $Img_{N+1}$. If the images have been previously registered to e.g. a previous reference image, the same transformation may be applied to all images $Img_{2-N}$. If unregistered images are stored in the buffer, a transformation may be determined for and applied to each image in the buffer separately;
4) computing a weight factor $w_{N+1}$ as described above and adding it to the buffer;
5) normalizing weight factors $w_2$ to $w_{N+1}$, e.g. according to equation (16):

$$w_k' = \frac{w_k}{\sum_{i=2}^{i=N+1}(w_i + \beta_1)} \qquad (16)$$

or according to equation (17):

$$w_k' = \frac{w_k}{\sum_{i=2}^{i=N+1}(w_i - w_{\min} + \beta_2)} \qquad (17)$$

Here, $\beta_1$ is a constant that can be positive or negative, $w_{\min}$ is defined as the minimum weight in the buffer and $\beta_2$ is a constant that should be greater than zero. To avoid negative weights and divisions by zero, any weight that is negative or zero can be set to a small positive number. The advantage of using the second normalization with $w_{\min}$ included is to increase the influence of the weight factor. Increasing $\beta_1$ or $\beta_2$ will decrease the influence of the weight factor and cause the algorithm to behave more like an averaging algorithm while decreasing $\beta_1$ or $\beta_2$ will increase the influence of the weight factor. These constants can be predetermined based on the application.

6) computing a high-quality combined image $Img_{N+1}'$ by computing a weighted average, using the previously computed weights, e.g. as defined in equation (18):

$$Img'_{N+1}(i,j) = \sum_{k=2}^{N+1} w' \cdot Img_k(i,j) \qquad (18)$$

Here, $i$ and $j$ are used to index the pixels for the images.

In an alternative embodiment, the image buffer may only comprise the combined image. In such an embodiment, a weighted average may be determined by using a buffer of N weight factors $w_k$, with k = 1, 2..., N corresponding to the N most recent images, and a combined image $Img_N'$ based on the N most recent images. For every new image $Img_{N+1}$ the following steps may be taken:

1) adding the new image to the buffer as $Img_{N+1}$, which may be selected as a reference image;
2) removing a first weight factor $w_1$ from the buffer;
3) applying a geometrical transformation to the stored image $Img_N'$ to register it to the reference image $Img_{N+1}$.
4) computing a weight factor $w_{N+1}$ as described above and adding it to the buffer;
5) normalizing weight factors $w_2$ to $w_{N+1}$, e.g. according to equation (16) or (17);
6) computing a high-quality combined image $Img_{N+1}'$ by computing a weighted average of the stored image $Img'_N$

and the reference image $\text{Img}_{N+1}$, e.g. as defined in equation (19):

$$\text{Img}'_{N+1}(i,j) = (1 - w_{N+1}) \cdot \text{Img}_N(i,j) + w_{N+1} \cdot \text{Img}_{N+1}(i,j) \qquad (19)$$

where $i$ and $j$ are used to index the pixels for the images; and
7) removing $\text{Img}_N$' from the buffer and adding $\text{Img}_{N+1}$' to the buffer.

The advantage of this algorithm is that it is much faster to compute since only one geometrical transformation has to be applied and the amount of processing steps is lower. The size of the buffer where the weight factors are stored determines how large the influence of the history images are compared to the influence of the new image. When the buffer is small, the new image will be more present in the final image while if the buffer is large the new image will be less present while images with a high weight factor will be more present.

As was explained above, the weights may be determined for an image as a whole, for each pixel in an image, or for predetermined or dynamically determined regions in an image. Similarly, a single transformations may be applied to each image as a whole, each pixel may be individually transformed, or the transformation may be determined for an applied to predetermined or dynamically determined regions. Thus, the weights may be defined as scalars, as matrices, or in different format.

An advantage of an algorithm using geometrical transformations based on e.g. rectangular or triangular meshes, and using weights determined per mesh segment, is that such an algorithm may be more robust to registration errors while still being able to correct locally for noise such as local motion.

A weight based on the amount of displacement or amount of transformation may be determined quickly for each image, independent of other images. Images with a large amount of displacement are generally noisier, and may therefore be assigned a lower weight, thus increasing the quality of the combined image.

Alternatively or additionally, a normalised amount of speckle contrast or an amount of change in speckle contrast relative to one or more previous and/or subsequent images in the sequence of first speckle contrast images may be determined for each first raw speckle image. In that case, the weighted average may be determined using weights based on the determined normalised amount of speckle contrast or the determined change in speckle contrast associated with the respective first speckle contrast image.

Weights based on differences or changes in speckle contrast, especially sudden changes, may be indicative for image quality. Typically, speckle contrast, and hence these weights, may be affected by various factors in the entire system, e.g. motion of the camera relative to the target area, movement of fibres or other factors influencing the optical path length, loose connections, or fluctuating lighting conditions. Hence, using weights based on speckle contrast, a higher quality combined image may be obtained. Typically, speckle contrast is determined in relative units, so weights may be determined by analysing a sequence of raw speckle images. As speckle contrast is inversely correlated with perfusion, speckle contrast-based perfusion units could similarly be used.

Preferably, the images may be normalized in such a way that the relation between the speckle contrast and weight is a linear with a constant. In that case, speckle-contrast-based correction could be more real-time, because each image might be normalized directly and without reference to a temporal window of images. Alternatively, an incremental average might be used.

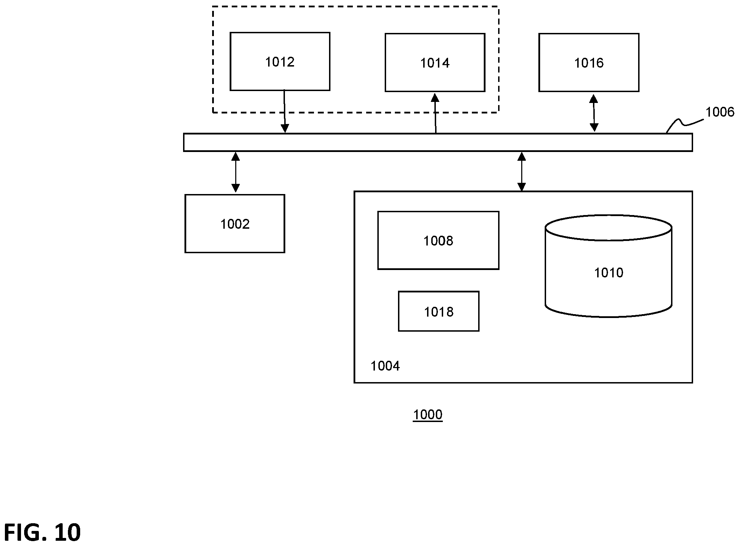

**Fig. 10** is a block diagram illustrating exemplary data processing systems described in this disclosure. Data processing system **1000** may include at least one processor **1002** coupled to memory elements **1004** through a system bus **1006**. As such, the data processing system may store program code within memory elements **1004**. Further, processor **1002** may execute the program code accessed from memory elements **1004** via system bus **1006**. In one aspect, data processing system may be implemented as a computer that is suitable for storing and/or executing program code. It should be appreciated, however, that data processing system **1000** may be implemented in the form of any system including a processor and memory that is capable of performing the functions described within this specification.

Memory elements **1004** may include one or more physical memory devices such as, for example, local memory **1008** and one or more bulk storage devices **1010**. Local memory may refer to random access memory or other non-persistent memory device(s) generally used during actual execution of the program code. A bulk storage device may be implemented as a hard drive or other persistent data storage device. The processing system **1000** may also include one or more cache memories (not shown) that provide temporary storage of at least some program code in order to reduce the number of times program code must be retrieved from bulk storage device **1010** during execution.

Input/output (I/O) devices depicted as key device **1012** and output device **1014** optionally can be coupled to the data processing system. Examples of key device may include, but are not limited to, for example, a keyboard, a pointing device such as a mouse, or the like. Examples of output device may include, but are not limited to, for example, a monitor or display, speakers, or the like. Key device and/or output device may be coupled to data processing system either directly or through intervening I/O controllers. A network adapter **1016** may also be coupled to data processing system to enable it to become coupled to other systems, computer systems, remote network devices, and/or remote storage devices through intervening private or public networks. The network adapter may comprise a data receiver for receiving data that is transmitted by said systems, devices and/or networks to said data and a data transmitter for transmitting data to said systems, devices and/or networks. Operation modems, cable operation modems, and Ethernet cards are examples of different types of network adapter that may be used with data processing system **1000**.

As pictured in **FIG. 10**, memory elements **1004** may store an application **1018**. It should be appreciated that data processing system **1000** may further execute an operating system (not shown) that can facilitate execution of the application. Application, being implemented in the form of executable program code, can be executed by data processing system **1000**, e.g., by processor **1002**. Responsive to executing application, data processing system may be configured to perform one or more operations to be described herein in further detail.

In one aspect, for example, data processing system **1000** may represent a client data processing system. In that case, application **1018** may represent a client application that, when executed, configures data processing system **1000** to perform the various functions described herein with reference to a "client". Examples of a client can include, but are not limited to, a personal computer, a portable computer, a mobile phone, or the like.

In another aspect, data processing system may represent a server. For example, data processing system may represent an (HTTP) server in which case application **1018**, when executed, may configure data processing system to perform (HTTP) server operations. In another aspect, data processing system may represent a module, unit or function as referred to in this specification.

The terminology used herein is for the purpose of describing particular embodiments only and is not intended to be limiting of the invention. As used herein, the singular forms "a," "an," and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

The invention is defined by the appended claims.

**Claims**

1. A method of motion-compensated laser speckle contrast imaging comprising:

   exposing (402) a target area to coherent first light of a first wavelength, the target area including living tissue;
   capturing (404) at least one sequence of images, the at least one sequence of images comprising first speckle images, the first speckle images being captured during the exposure with the first light;
   determining (406) one or more transformation parameters of an image registration algorithm for registering the first speckle images with each other, the transformation parameters being based on a similarity measure of pixel values of at least one group of pixels in each of a plurality of images of the at least one sequence of images, the images in the plurality of images being selected from the first speckle images or being associated with the first speckle images; determining first speckle contrast images based on the first speckle images;
   determining (408) registered speckle contrast images by registering the first speckle contrast images based on the one or more transformation parameters and the image registration algorithm; and
   computing a weighted average of the registered first speckle contrast images.

2. The method as claimed in claim 1, the method further comprising:

   exposing the target area to second light of one or more second wavelengths, preferably coherent light of a second wavelength or light comprising a plurality of second wavelengths of the visible spectrum, wherein the exposure to the second light is alternated with the exposure to the first light or is simultaneous with the exposure to the first light;
   wherein the at least one sequence of images comprises a sequence of second images, the second images being captured during exposure with the second light; and
   wherein the plurality of images is selected from the sequence of second images, each of the second images being associated with a first speckle image.

3. The method as claimed in claim 2, wherein the light of the at least second wavelength is coherent light of a predetermined second wavelength, preferably in the green or blue part of the electromagnetic spectrum, preferably in the range 380-590 nm, more preferably in the range 470-570 nm, even more preferably in the range 520-560 nm, and wherein the sequence of second images is a sequence of second speckle images;
the method further comprising:

determining second speckle contrast images based on the sequence of second speckle images; and
adjusting the first speckle contrast images based on changes in speckle contrast magnitude in the sequence of second speckle contrast images.

4. The method as claimed in any of the preceding claims, wherein the first wavelength is a wavelength in the red part of the electromagnetic spectrum, preferably in the range 600-700 nm, more preferably in the range 620-660 nm, or in the infrared part of the electromagnetic spectrum, preferably in the range 700-1200 nm.

5. The method as claimed in any of the preceding claims, wherein a weight of an image is based on the transformation parameters or based on a relative magnitude of the speckle contrast.

6. The method as claimed in any of the preceding claims, wherein the groups of pixels represent predetermined features in the plurality of images, the predetermined features preferably being associated with objects, preferably anatomical structures, in the target area.

7. The method as claimed in any of the preceding claims, wherein determining one or more transformation parameters comprises:

determining a plurality of associated groups of pixels based on the similarity measure, each group of pixels belonging to a different image from the plurality of images,
determining a plurality of displacement vectors based on positions of the groups of pixels relative to the respective images from the plurality of images, the displacement vectors representing motion of the target area relative to the image sensor; and
determining the transformation parameters based on the plurality of displacement vectors.

8. The method as claimed in any of the preceding claims, further comprising:

dividing each image of the first speckle images, respectively first speckle contrast images, and each image in the plurality of images into a plurality of regions, preferably disjoint regions; and wherein
determining transformation parameters comprises determining transformation parameters for each region; and
determining a sequence of registered first speckle images, respectively first speckle contrast images comprises registering each region of the first speckle image, respectively first speckle contrast image, based on the transformation based on the corresponding region in the second image.

9. The method as claimed in any of the preceding claims, wherein the target area comprises a perfused organ, preferably perfused by a bodily fluid, more preferably perfused by blood and/or lymph fluid, and/or comprises on or more blood vessels and/or lymphatic vessels, the method further comprising:
computing a perfusion intensity, preferably a blood perfusion intensity or a lymph perfusion intensity, based on the combined speckle image.

10. The method as claimed in any of the preceding claims, further comprising displaying the combined speckle contrast image or a derivative thereof.

11. A hardware module for an imaging device, preferably a medical imaging device, comprising:

a first light source (104) for exposing a target area (102) to coherent first light of a first wavelength, the target area (102) including living tissue;
at least one image sensor system (108) for capturing at least one sequence of images, the at least one sequence of images comprising first speckle images, the first speckle images being captured during the exposure with the first light;
a computer readable storage medium having computer readable program code embodied therewith, and a processor, preferably a microprocessor, more preferably a graphics processing unit, coupled to the computer readable storage medium, wherein responsive to executing the computer readable program code, the processor is configured to:

determine one or more transformation parameters of an image registration algorithm for registering the first speckle images with each other, the transformation parameters being based on a similarity measure of pixel values of at least one group of pixels in each of a plurality of images, the images in the plurality of images being selected from the first laser speckle images or being associated with the first speckle images; determine first speckle contrast images based on the first speckle images determine registered speckle contrast images by registering the first speckle contrast images based on the one or more transformation parameters and the image registration algorithm; and compute a weighted average of the registered first speckle contrast images.

12. The hardware module as claimed in claim 11, further comprising:

a second light source (104) for illuminating, simultaneous or alternatingly with the first light source, the target area (102) with light of at least a second wavelength, different from the first wavelength; wherein the at least one image sensor (108) is further configured to capture a sequence of second images, the second images being captured during exposure with the second light; and wherein the plurality of images is selected from the sequence of second images, each of the second images being associated with a first speckle image.

13. The hardware module as claimed in claim 11 or 12, further comprising: a display for displaying the combined speckle image and/or a derivative thereof, preferably a perfusion intensity image.

14. A medical imaging device (100) comprising a hardware module according to any of claims 11-13, the device preferably being one of: endoscope, a laparoscope, a surgical robot, a handheld laser speckle contrast imaging device or an open surgical laser speckle contrast imaging system.

15. A computation module (110) for a laser speckle imaging system, comprising a computer readable storage medium having at least a part of a program embodied therewith, and a processor, preferably a microprocessor, more preferably a graphics processing unit, coupled to the computer readable storage medium, wherein responsive to executing the computer readable storage code, the processor is configured to perform executable operations, the executable operations comprising:

receiving at least one sequence of images, the at least one sequence of images comprising first speckle images, the first speckle images having been captured during exposure of a target area to coherent first light of a first wavelength, the target area including living tissue; determining one or more transformation parameters of an image registration algorithm for registering the first speckle images with each other, the transformation parameters being based on a similarity measure of pixel values of at least on group of pixels in each of a plurality of images of the at least one sequence of images, the images in the plurality of images being selected from the first speckle images or being associated with the first speckle images; determining first speckle contrast images based on the first speckle images; determining registered speckle contrast images by registering the first speckle contrast images based on the one or more transformation parameters and the image registration algorithm; and computing a weighted average of the registered first speckle contrast images.

16. The computation module (110) as claimed in claim 15,

wherein the at least one sequence of images comprises a sequence of second images, the second images being captured during exposure with second light, the second light having one or more second wavelengths, preferably the second light being coherent light of a second wavelength or the second light comprising a plurality of second wavelengths of the visible spectrum, wherein the exposure to the second light is alternated with the exposure to the first light or is simultaneous with the exposure to the first light; and wherein the plurality of images is selected from the sequence of second images, each of the second images being associated with a first speckle image.

17. A computer program or suite of computer programs comprising at least one software code portion or a computer program product storing at least one software code portion, the software code portion, when run on a computer system, being configured for executing the method of motion-compensated laser speckle contrast imaging comprising: determining (406) one or more transformation parameters of an image registration algorithm for registering first speckle images with each other, the transformation parameters being based on a similarity measure of pixel values of at least one group of pixels in each of a plurality of images of at least one sequence of images, the images in the plurality of images being selected from the first speckle images or being associated with the first speckle images; determining first speckle contrast images based on the first speckle images;determining (408) registered speckle contrast images by registering the first speckle contrast images based on the one or more transformation parameters and the image registration algorithm; and computing a weighted average of the registered first speckle contrast images.

## Patentansprüche

1. Verfahren zur bewegungskompensierten Laser-Speckle-Kontrast-Bildgebung, das aufweist:

   Belichten (402) eines Zielgebiets mit kohärentem ersten Licht einer ersten Wellenlänge, wobei der Zielgebiet lebendes Gewebe enthält;
   Erfassen (404) zumindest einer Bildersequenz, wobei die zumindest eine Bildersequenz erste Speckle-Bilder aufweist, wobei die ersten Speckle-Bilder während der Belichtung mit dem ersten Licht erfasst werden;
   Bestimmen (406) eines oder mehrerer Transformationsparameter eines Bildregistrierungsalgorithmus zum Registrieren der ersten Speckle-Bilder miteinander, wobei die Transformationsparameter auf einem Ähnlichkeitsmaß von Pixelwerten zumindest einer Gruppe von Pixeln in jedem einer Mehrzahl von Bildern der zumindest einen Bildersequenz basieren, wobei die Bilder in der Mehrzahl von Bildern aus den ersten Speckle-Bildern ausgewählt werden oder mit den ersten Speckle-Bildern assoziiert werden;
   Bestimmen (408) registrierter Speckle-Kontrastbilder durch Registrieren der ersten Speckle-Kontrastbilder basierend auf dem einen oder den mehreren Transformationsparametern und dem Bildregistrierungsalgorithmus; und
   Berechnen eines gewichteten Durchschnitts der registrierten ersten Speckle-Kontrastbilder.

2. Verfahren nach Anspruch 1, wobei das Verfahren weiterhin aufweist:

   Belichten des Zielgebiets mit zweitem Licht einer oder mehrerer zweiter Wellenlängen, vorzugsweise kohärentem Licht einer zweiten Wellenlänge oder Licht, das eine Mehrzahl von zweiten Wellenlängen des sichtbaren Spektrums aufweist, wobei die Belichtung mit dem zweiten Licht abwechselnd mit der Belichtung mit dem ersten Licht oder gleichzeitig mit der Belichtung mit dem ersten Licht erfolgt;
   wobei die zumindest eine Bildersequenz eine Sequenz von zweiten Bildern aufweist, wobei die zweiten Bilder während der Belichtung mit dem zweiten Licht erfasst werden; und
   wobei die Mehrzahl von Bildern aus der Sequenz von zweiten Bildern ausgewählt wird, wobei jedes der zweiten Bilder mit einem ersten Speckle-Bild assoziiert wird.

3. Verfahren nach Anspruch 2, wobei das Licht der zumindest einen zweiten Wellenlänge kohärentes Licht einer vorbestimmten zweiten Wellenlänge ist, vorzugsweise im grünen oder blauen Teil des elektromagnetischen Spektrums, vorzugsweise im Bereich 380-590 nm, noch bevorzugter im Bereich 470-570 nm, noch bevorzugter im Bereich 520-560 nm, und wobei die Sequenz von zweiten Bildern eine Sequenz von zweiten Speckle-Bildern ist;
   wobei das Verfahren weiterhin aufweist:

   Bestimmen von zweiten Speckle-Kontrastbildern basierend auf der Sequenz von zweiten Speckle-Bildern; und
   Einstellen der ersten Speckle-Kontrastbilder basierend auf Änderungen der Speckle-Kontraststärke in der Sequenz der zweiten Speckle-Kontrastbilder.

4. Verfahren nach einem der vorhergehenden Ansprüche, wobei die erste Wellenlänge eine Wellenlänge im roten Teil des elektromagnetischen Spektrums ist, vorzugsweise im Bereich 600-700 nm, bevorzugter im Bereich 620-660 nm, oder im Infrarot-Teil des elektromagnetischen Spektrums, vorzugsweise im Bereich 700-1200 nm.

5. Verfahren nach einem der vorhergehenden Ansprüche, wobei eine Gewichtung eines Bildes auf den Transformationsparametern oder auf einer relativen Stärke des Speckle-Kontrasts beruht.

6. Verfahren nach einem der vorhergehenden Ansprüche, wobei die Gruppen von Pixeln vorbestimmte Merkmale in der Mehrzahl von Bildern darstellen, wobei die vorbestimmten Merkmale vorzugsweise mit Objekten, vorzugsweise anatomischen Strukturen, in dem Zielgebiet assoziiert werden.

7. Verfahren nach einem der vorhergehenden Ansprüche, wobei das Bestimmen eines oder mehrerer Transformationsparameter aufweist:

Bestimmen einer Mehrzahl von assoziierten Gruppen von Pixeln auf der Grundlage des Ähnlichkeitsmaßes, wobei jede Gruppe von Pixeln zu einem anderen Bild aus der Mehrzahl von Bildern gehört,
Bestimmen einer Mehrzahl von Verschiebungsvektoren basierend auf Positionen der Gruppen von Pixeln relativ zu den jeweiligen Bildern aus der Mehrzahl von Bildern, wobei die Verschiebungsvektoren eine Bewegung des Zielgebiets relativ zum Bildsensor darstellen; und
Bestimmen der Transformationsparameter basierend auf der Mehrzahl von Verschiebungsvektoren.

8. Verfahren nach einem der vorhergehenden Ansprüche, das weiterhin aufweist:

Aufteilen jedes Bildes der ersten Speckle-Bilder bzw. der ersten Speckle-Kontrastbilder und jedes Bildes in der Mehrzahl von Bildern in eine Mehrzahl von Regionen, vorzugsweise disjunkte Regionen; und wobei
das Bestimmen von Transformationsparametern das Bestimmen von Transformationsparametern für jede Region aufweist; und
das Bestimmen einer Sequenz von registrierten ersten Speckle-Bildern bzw. ersten Speckle-Kontrastbildern ein Registrieren jeder Region des ersten Speckle-Bildes bzw. ersten Speckle-Kontrastbildes basierend auf der Transformation, die auf der entsprechenden Region im zweiten Bild basiert, aufweist.

9. Verfahren nach einem der vorhergehenden Ansprüche, wobei das Zielgebiet ein perfundiertes Organ aufweist, das vorzugsweise von einer Körperflüssigkeit perfundiert wird, bevorzugter von Blut und/oder Lymphflüssigkeit perfundiert wird, und/oder ein oder mehrere Blutgefäße und/oder Lymphgefäße aufweist, wobei das Verfahren weiterhin aufweist:
Berechnen einer Perfusionsintensität, vorzugsweise einer Blutperfusionsintensität oder einer Lymphperfusionsintensität, basierend auf dem kombinierten Speckle-Bild.

10. Verfahren nach einem der vorhergehenden Ansprüche, das weiterhin Anzeigen des kombinierten Speckle-Kontrastbildes oder eines Derivats davon aufweist.

11. Hardwaremodul für eine Bildgebungsvorrichtung, vorzugsweise eine medizinische Bildgebungsvorrichtung, das aufweist:

eine erste Lichtquelle (104) zum Belichten eines Zielgebiets (102) mit kohärentem ersten Licht einer ersten Wellenlänge, wobei das Zielgebiet (102) lebendes Gewebe enthält;
zumindest ein Bildsensorsystem (108) zum Erfassen zumindest einer Bildersequenz, wobei die zumindest eine Bildersequenz erste Speckle-Bilder umfasst, wobei die ersten Speckle-Bilder während der Belichtung mit dem ersten Licht erfasst werden;
ein computerlesbares Speichermedium, das einen computerlesbaren Programmcode enthält, und einen Prozessor, vorzugsweise einen Mikroprozessor, bevorzugter eine Grafikverarbeitungseinheit, der mit dem computerlesbaren Speichermedium verbunden ist, wobei der Prozessor in Reaktion auf die Ausführung des computerlesbaren Programmcodes konfiguriert ist, um:

einen oder mehrere Transformationsparameter eines Bildregistrierungsalgorithmus zu bestimmen, um die ersten Speckle-Bilder miteinander zu registrieren, wobei die Transformationsparameter auf einem Ähnlichkeitsmaß von Pixelwerten zumindest einer Gruppe von Pixeln in jedem einer Mehrzahl von Bildern basieren, wobei die Bilder in der Mehrzahl von Bildern aus den ersten Laser-Speckle-Bildern ausgewählt werden oder mit den ersten Speckle-Bildern assoziiert werden;
erste Speckle-Kontrastbilder basierend auf den ersten Speckle-Bildern zu bestimmen;
registrierte Speckle-Kontrastbilder durch Registrieren der ersten Speckle-Kontrastbilder basierend auf dem einen oder den mehreren Transformationsparametern und des Bildregistrierungsalgorithmus zu bestimmen; und
einen gewichteten Durchschnitt der registrierten ersten Speckle-Kontrastbilder zu berechnen.

12. Hardwaremodul nach Anspruch 11, das weiterhin aufweist:

eine zweite Lichtquelle (104) zum Belichten, gleichzeitig oder abwechselnd mit der ersten Lichtquelle, des Zielgebiets (102) mit Licht zumindest einer zweiten Wellenlänge, die von der ersten Wellenlänge verschieden ist; wobei der zumindest eine Bildsensor (108) weiterhin konfiguriert ist, um eine Sequenz von zweiten Bildern aufzunehmen, wobei die zweiten Bilder während der Belichtung mit dem zweiten Licht erfasst werden; und wobei die Mehrzahl von Bildern aus der Sequenz von zweiten Bildern ausgewählt wird, wobei jedes der zweiten Bilder mit einem ersten Speckle-Bild assoziiert wird.

**13.** Hardwaremodul nach Anspruch 11 oder 12, das weiterhin aufweist:
ein Display zum Anzeigen des kombinierten Speckle-Bildes und/oder eines Derivativs davon, vorzugsweise eines Perfusionsintensitätsbildes.

**14.** Medizinische Bildgebungsvorrichtung (100), die ein Hardwaremodul nach einem der Ansprüche 11-13 aufweist, wobei die Vorrichtung vorzugsweise eines von folgendem ist: ein Endoskop, ein Laparoskop, ein chirurgischer Roboter, eine handgehaltene Laser-Speckle-Kontrast-Bildgebungsvorrichtung, oder ein Laser-Speckle-Kontrast-Bildgebungssystem für offene Chirurgie.

**15.** Berechnungsmodul (110) für ein Laser-Speckle-Bildgebungssystem, das ein computerlesbares Speichermedium, das zumindest einen Teil eines Programms enthält, und einen Prozessor aufweist, vorzugsweise einen Mikroprozessor, bevorzugter eine Grafikverarbeitungseinheit, der mit dem computerlesbaren Speichermedium verbunden ist, wobei der Prozessor in Reaktion auf die Ausführung des computerlesbaren Speichercodes konfiguriert ist, um ausführbare Operationen durchzuführen, wobei die ausführbaren Operationen umfassen:

Empfangen zumindest einer Bildersequenz, wobei die zumindest eine Bildersequenz erste Speckle-Bilder aufweist, wobei die ersten Speckle-Bilder während der Belichtung eines Zielgebiets mit kohärentem ersten Licht einer ersten Wellenlänge erfasst wurden, wobei das Zielgebiet lebendes Gewebe enthält;
Bestimmen eines oder mehrerer Transformationsparameter eines Bildregistrierungsalgorithmus zum Registrieren der ersten Speckle-Bilder miteinander, wobei die Transformationsparameter auf einem Ähnlichkeitsmaß von Pixelwerten von zumindest einer Gruppe von Pixeln in jedem einer Mehrzahl von Bildern der zumindest einen Bildersequenz basieren, wobei die Bilder in der Mehrzahl von Bildern aus den ersten Speckle-Bildern ausgewählt oder mit den ersten Speckle-Bildern assoziiert werden;
Bestimmen erster Speckle-Kontrastbilder basierend auf den ersten Speckle-Bildern;
Bestimmen registrierter Speckle-Kontrastbilder durch Registrieren der ersten Speckle-Kontrastbilder basierend auf dem einen oder den mehreren Transformationsparametern und dem Bildregistrierungsalgorithmus; und
Berechnen eines gewichteten Durchschnitts der registrierten ersten Speckle-Kontrastbilder.

**16.** Berechnungsmodul (110) nach Anspruch 15,

wobei die zumindest eine Bildersequenz eine Sequenz von zweiten Bildern aufweist, wobei die zweiten Bilder während einer Belichtung mit zweitem Licht erfasst werden, wobei das zweite Licht eine oder mehrere zweite Wellenlängen aufweist, vorzugsweise wobei das zweite Licht kohärentes Licht einer zweiten Wellenlänge ist oder das zweite Licht eine Mehrzahl von zweiten Wellenlängen des sichtbaren Spektrums aufweist, wobei die Belichtung mit dem zweiten Licht mit der Belichtung mit dem ersten Licht abwechselt oder gleichzeitig mit der Belichtung mit dem ersten Licht erfolgt; und
wobei die Mehrzahl von Bildern aus der Sequenz von zweiten Bildern ausgewählt wird, wobei jedes der zweiten Bilder mit einem ersten Speckle-Bild assoziiert wird.

**17.** Computerprogramm oder eine Reihe von Computerprogrammen mit zumindest einem Softwarecodeabschnitt oder ein Computerprogrammprodukt, das zumindest einen Softwarecodeabschnitt speichert, wobei der Softwarecodeabschnitt, wenn er auf einem Computersystem ausgeführt wird, konfiguriert ist, um das Verfahren einer bewegungskompensierten Laser-Speckle-Kontrast-Bildgebung auszuführen, das aufweist:
Bestimmen (406) eines oder mehrerer Transformationsparameter eines Bildregistrierungsalgorithmus zum Registrieren von ersten Speckle-Bildern miteinander, wobei die Transformationsparameter auf einem Ähnlichkeitsmaß von Pixelwerten zumindest einer Gruppe von Pixeln in jedem einer Mehrzahl von Bildern zumindest einer Bildersequenz basieren, wobei die Bilder in der Mehrzahl von Bildern aus den ersten Speckle-Bildern ausgewählt werden oder mit den ersten Speckle-Bildern assoziiert werden; Bestimmen erster Speckle-Kontrastbilder basierend auf den ersten Speckle-Bildern; Bestimmen (408) registrierter Speckle-Kontrastbilder durch Registrieren der ersten Speckle-Kontrastbilder basierend auf dem einen oder den mehreren Transformationsparametern und dem Bildregistrierungsalgorithmus; und Berechnen eines gewichteten Durchschnitts der registrierten ersten Speckle-Kontrastbilder.

**Revendications**

1. Procédé d'imagerie de contraste par speckle laser à compensation de mouvement comprenant :

   l'exposition (402) d'une zone cible à une première lumière cohérente d'une première longueur d'onde, la zone cible incluant des tissus vivants ;
   la capture (404) d'au moins une séquence d'images, l'au moins une séquence d'images comprenant des premières images de speckle, les premières images de speckle étant capturées pendant l'exposition à la première lumière ;
   la détermination (406) d'un ou de plusieurs paramètres de transformation d'un algorithme de recalage d'images pour recaler les premières images de speckle les unes avec les autres, les paramètres de transformation étant basés sur une mesure de similarité de valeurs de pixel d'au moins un groupe de pixels dans chacune d'une pluralité d'images de l'au moins une séquence d'images, les images dans la pluralité d'images étant sélectionnées parmi les premières images de speckle ou étant associées aux premières images de speckle ; la détermination de premières images de contraste par speckle étant basée sur les premières images de speckle ;
   la détermination (408) d'images de contraste par speckle recalées par recalage des premières images de contraste par speckle sur la base des un ou plusieurs paramètres de transformation et de l'algorithme de recalage d'images ; et
   le calcul d'une moyenne pondérée des premières images de contraste par speckle recalées.

2. Procédé selon la revendication 1, le procédé comprenant en outre :

   l'exposition de la zone cible à une seconde lumière d'une ou plusieurs longueurs d'onde, de préférence à une lumière cohérente d'une seconde longueur d'onde ou à une lumière comprenant une pluralité de secondes longueurs d'onde du spectre visible, dans lequel l'exposition à la seconde lumière est alternée avec l'exposition à la première lumière ou est simultanée à l'exposition à la première lumière ;
   dans lequel l'au moins une séquence d'images comprend une séquence de secondes images, les secondes images étant capturées pendant l'exposition à la seconde lumière ; et
   dans lequel la pluralité d'images sont sélectionnées parmi la séquence de secondes images, chacune des secondes images étant associée à une première image de speckle.

3. Procédé selon la revendication 2, dans lequel la lumière de l'au moins seconde longueur d'onde est une lumière cohérente d'une seconde longueur d'onde prédéterminée, de préférence dans la partie verte ou bleue du spectre électromagnétique, de préférence dans la plage de 380-590 nm, de manière davantage préférée dans la plage de 470-570 nm, de manière encore davantage préférée dans la plage de 520-560 nm, et dans lequel la séquence de secondes images est une séquence de secondes images de speckle ;
   le procédé comprenant en outre :

   la détermination de secondes images de contraste par speckle sur la base de la séquence de secondes images de speckle ; et
   l'ajustement des premières images de contraste par speckle sur la base de variations d'une amplitude de contraste par speckle dans la séquence de secondes images de contraste par speckle.

4. Procédé selon l'une quelconque des revendications précédentes, dans lequel la première longueur d'onde est une longueur d'onde dans la partie rouge du spectre électromagnétique, de préférence dans la plage de 600-700 nm, de manière davantage préférée dans la plage de 620-660 nm, ou dans la partie infrarouge du spectre électromagnétique, de préférence dans la plage de 700-1 200 nm.

5. Procédé selon l'une quelconque des revendications précédentes, dans lequel un poids d'une image est basé sur les paramètres de transformation ou basé sur une amplitude relative du contraste par speckle.

6. Procédé selon l'une quelconque des revendications précédentes, dans lequel les groupes de pixels représentent des caractéristiques prédéterminées dans la pluralité d'images, les caractéristiques prédéterminées étant de préférence associées à des objets, de préférence à des structures anatomiques, dans la zone cible.

7. Procédé selon l'une quelconque des revendications précédentes, dans lequel la détermination d'un ou de plusieurs paramètres de transformation comprend :

la détermination d'une pluralité de groupes associés de pixels sur la base de la mesure de similarité, chaque groupe de pixels appartenant une image différente de la pluralité d'images,

la détermination d'une pluralité de vecteurs de déplacement sur la base de positions des groupes de pixels par rapport aux images respectives parmi la pluralité d'images, les vecteurs de déplacement représentant un mouvement de la zone cible par rapport au capteur d'images ; et

la détermination des paramètres de transformation sur la base de la pluralité de vecteurs de déplacement.

**8.** Procédé selon l'une quelconque des revendications précédentes, comprenant en outre :

la division de chaque image des premières images de speckle, respectivement des premières images de contraste par speckle, et de chaque image dans la pluralité d'images en une pluralité de régions, de préférence en régions disjointes ; et dans lequel

la détermination de paramètres de transformation comprend la détermination de paramètres de transformation pour chaque région ; et

la détermination d'une séquence de premières images de speckle recalées, respectivement de premières images de contraste par speckle comprend le recalage de chaque région de la première image de speckle, respectivement la première image de contraste par speckle, sur la base de la transformation basée sur la région correspondante dans la seconde image.

**9.** Procédé selon l'une quelconque des revendications précédentes, dans lequel la zone cible comprend un organe perfusé, de préférence perfusé par un fluide corporel, de manière davantage préférée perfusé par du sang et/ou du liquide lymphatique, et/ou comprend un ou plusieurs vaisseaux sanguins et/ou vaisseaux lymphatiques, le procédé comprenant en outre :

le calcul d'une intensité de perfusion, de préférence une intensité de perfusion sanguine ou de perfusion lymphatique, sur la base de l'image de speckle combinée.

**10.** Procédé selon l'une quelconque des revendications précédentes, comprenant en outre l'affichage de l'image de contraste par speckle combinée ou d'un dérivé de celle-ci.

**11.** Module matériel pour un dispositif d'imagerie, de préférence un dispositif d'imagerie médicale, comprenant :

une première source de lumière (104) pour exposer une zone cible (102) à une première lumière cohérente d'une première longueur d'onde, la zone cible (102) incluant des tissus vivants ;

au moins un système de capteur d'images (108) pour capturer au moins une séquence d'images, l'au moins une séquence d'images comprenant des premières images de speckle, les premières images de speckle étant capturées pendant l'exposition à la première lumière ;

un support de stockage lisible par ordinateur présentant un code de programme lisible par ordinateur intégré dans celui-ci, et un processeur, de préférence un microprocesseur, de manière davantage préférée une unité de traitement graphique, couplé au support de stockage lisible par ordinateur, dans lequel en réponse à l'exécution du code de programme lisible par ordinateur, le processeur est configuré pour :

déterminer un ou plusieurs paramètres de transformation d'un algorithme de recalage d'images pour recaler les premières images de speckle les unes avec les autres, les paramètres de transformation étant basés sur une mesure de similarité de valeurs de pixel d'au moins un groupe de pixels dans chacune d'une pluralité d'images, les images de la pluralité d'images étant sélectionnées parmi les premières images de speckle laser ou étant associées aux premières images de speckle ;

déterminer des premières images de contraste par speckle sur la base des premières images de speckle déterminer des images de contraste par speckle recalées par recalage des premières images de contraste par speckle sur la base des un ou plusieurs paramètres de transformation et de l'algorithme de recalage d'images ; et

calculer une moyenne pondérée des premières images contraste par speckle recalées.

**12.** Module matériel selon la revendication 11, comprenant en outre :

une seconde source de lumière (104) pour éclairer, simultanément ou en alternance avec la première source de lumière, la zone cible (102) avec une lumière d'au moins une seconde longueur d'onde, différente de la première longueur d'onde ;

dans lequel l'au moins un capteur d'images (108) est configuré pour capturer une séquence de secondes images, les secondes images étant capturées pendant l'exposition à la seconde lumière ; et
dans lequel la pluralité d'images sont sélectionnées parmi la séquence de secondes images, chacune des secondes images étant associée à une première image de speckle.

13. Module matériel selon la revendication 11 ou 12, comprenant en outre :
un écran pour afficher l'image de speckle combinée et/ou un dérivé de celle-ci, de préférence une image d'intensité de perfusion.

14. Dispositif d'imagerie médicale (100) comprenant un module matériel selon l'une quelconque des revendications 11-13, le dispositif étant de préférence l'un parmi : un endoscope, un laparoscope, un robot chirurgical, un dispositif d'imagerie de contraste par speckle laser portatif ou un système d'imagerie de contraste par speckle laser chirurgicale ouverte.

15. Module de calcul (110) pour un système d'imagerie par speckle laser, comprenant un support de stockage lisible par ordinateur présentant au moins une partie d'un programme intégrée dans celui-ci, et un processeur, de préférence un microprocesseur, de manière davantage préférée une unité de traitement graphique, couplé au support de stockage lisible par ordinateur, dans lequel en réponse à l'exécution du code de stockage lisible par ordinateur, le processeur est configuré pour réaliser des opérations exécutables, les opérations exécutables comprenant :

la réception d'au moins une séquence d'images, l'au moins une séquence d'images comprenant des premières images de speckle, les premières images de speckle ayant été capturées pendant l'exposition d'une zone cible à une première lumière cohérente d'une première longueur d'onde, la zone cible incluant des tissus vivants ; la détermination d'un ou de plusieurs paramètres de transformation d'un algorithme de recalage d'images pour recaler les premières images de speckle les unes avec les autres, les paramètres de transformation étant basés sur une mesure de similarité de valeurs de pixel d'au moins un groupe de pixels dans chacune d'une pluralité d'images de l'au moins une séquence d'images, les images dans la pluralité d'images étant sélectionnées parmi les premières images de speckle ou étant associées aux premières images de speckle ; la détermination de premières images de contraste par speckle sur la base des premières images de speckle ; la détermination d'images de contraste par speckle recalées par recalage des premières images contraste par speckle sur la base des un ou plusieurs paramètres de transformation et de l'algorithme de recalage d'images ; et le calcul d'une moyenne pondérée des premières images de contraste par speckle recalées.

16. Module de calcul (110) selon la revendication 15,

dans lequel l'au moins une séquence d'images comprend une séquence de secondes images, les secondes images étant capturées pendant l'exposition à une seconde lumière, la seconde lumière présentant une ou plusieurs secondes longueurs d'onde, de préférence la seconde lumière étant une lumière cohérente d'une seconde longueur d'onde ou la seconde lumière comprenant une pluralité de secondes longueurs d'onde du spectre visible, dans lequel l'exposition à la seconde lumière est alternée avec l'exposition à la première lumière ou est simultanée à l'exposition à la première lumière ; et
dans lequel la pluralité d'images sont sélectionnées parmi la séquence de secondes images, chacune des secondes images étant associée à une première image de speckle.

17. Programme informatique ou suite de programmes informatiques comprenant au moins une partie de code logiciel ou un produit-programme informatique stockant au moins une partie de code logiciel, la partie de code logiciel, lorsqu'elle est exécutée sur un système informatique, étant configurée pour exécuter le procédé d'imagerie de contraste par speckle laser à compensation de mouvement comprenant :
la détermination (406) d'un ou de plusieurs paramètres de transformation d'un algorithme de recalage d'images pour recaler des premières images de speckle les unes avec les autres, les paramètres de transformation étant basés sur une mesure de similarité de valeurs de pixel d'au moins un groupe de pixels dans chacune d'une pluralité d'images d'au moins une séquence d'images, les images dans la pluralité d'images étant sélectionnées parmi les premières images de speckle ou étant associée aux premières images de speckle ; la détermination de premières images de contraste par speckle sur la base des premières images de speckle ; la détermination (408) d'images de contraste par speckle recalées par recalage des premières images de contraste par speckle sur la base des un ou plusieurs paramètres de transformation et de l'algorithme de recalage d'images ; et le calcul du moyenne pondérée des premières images de contraste par speckle recalées.

FIG. 1A

FIG. 1B

**FIG. 1C**

**FIG. 1D**

speckle contrast images, before motion correction

$220_1$     $220_2$     $220_3$     $220_4$     $220_5$

speckle contrast images, after motion correction

$222_1$     $222_2$     $222_3$     $222_4$     $222_5$

**Fig. 2B**

EP 4 213 705 B1

Fig. 2C

EP 4 213 705 B1

$t = t_1$

single wavelength

302 — obtain 1st speckle image — 316

312

304 — calculate 1st speckle contrast image

$t = t_2$

single wavelength

312 — obtain 2nd speckle image — 316

314 — calculate 2nd speckle contrast image

308 — detect 1st features

318 — detect 2nd features

320 — determine displacement vectors

322 — determine transformation

324 — realign 1st speckle contrast image

326 — combine reregistered 1st and 2nd speckle contrast images

**FIG. 3A**

$t = t_1$

first wavelength                second wavelength

332

obtain 1st speckle image        obtain 1st correction image        336

334

calculate 1st speckle
contrast image

detect 1st features        338

$t = t_2$

342        first wavelength                second wavelength

obtain 2nd speckle image        obtain 2nd correction image        346

344

calculate 2nd speckle
contrast image

detect 2nd features        348

determine displacement
vectors        350

354

realign 1st speckle
contrast image

determine transformation        352

356

combine reregistered 1st and
2nd speckle contrast images

**FIG. 3B**

EP 4 213 705 B1

402 — exposing a target area to coherent light of a predetermined wavelength, the target area including living tissue

404 — capturing at least one sequence of images, the at least one sequence of images comprising speckle images, the speckle images being captured during the exposure with the coherent light

406 — determining one or more transformation parameters of an image registration algorithm for registering the speckle images with each other, the transformation parameters being based on a similarity measure of pixel values of groups of pixels in a plurality of images of the at least one sequence of images, the plurality of images being selected from the speckle images

408 — determining registered speckle images by registering the speckle images based on the one or more transformation parameters and the image registration algorithm, and determining a combined speckle contrast image based on the registered speckle images

410 — determining speckle contrast images based on the speckle images, determining registered speckle contrast images by registering the speckle contrast images based on the one or more transformation parameters and the image registration algorithm, and determining a combined speckle contrast image based on the registered speckle contrast images

**FIG. 4A**

EP 4 213 705 B1

```
┌────────────────────────────────────────────────────────────────────────────────────────────┐
│  alternatingly or simultaneously exposing a target area to coherent first light of a first   │
│  wavelength and to second light of one or more second wavelengths, preferably coherent       │
│  light of a second wavelength or light comprising a plurality of second wavelengths of the   │
│  visible spectrum, the target area including living tissue                                    │
└────────────────────────────────────────────────────────────────────────────────────────────┘
```
412

```
┌────────────────────────────────────────────────────────────────────────────────────────────┐
│  capturing a sequence of first speckle images during the exposure with the first light and   │
│  a sequence of second images during the exposure with the second light, a speckle image of   │
│  the sequence of first speckle images being associated with an image of the sequence of      │
│  second images                                                                                │
└────────────────────────────────────────────────────────────────────────────────────────────┘
```
414

```
┌────────────────────────────────────────────────────────────────────────────────────────────┐
│  determining one or more transformation parameters of a registration algorithm for           │
│  registering at least a part of the sequence of first speckle images based on a similarity    │
│  measure of pixel values of groups of pixels in at least a part of the sequence of second     │
│  images associated with the first speckle images                                              │
└────────────────────────────────────────────────────────────────────────────────────────────┘
```
416

418

```
┌─────────────────────────────────────────┐
│  determining registered first speckle    │
│  images by registering the at least      │
│  part of the sequence of first speckle   │
│  images based on the one or more         │
│  transformation parameters and the       │
│  registration algorithm, and             │
│  determining a combined speckle          │
│  contrast image based on the             │
│  registered first speckle images         │
└─────────────────────────────────────────┘
```

420

```
┌─────────────────────────────────────────┐
│  determining first speckle contrast      │
│  images based on the at least part of    │
│  the sequence of first speckle images,   │
│  determining registered speckle          │
│  contrast images by registering the      │
│  first speckle contrast images based     │
│  on the one or more transformation       │
│  parameters and the registration         │
│  algorithm, and determining a combined   │
│  speckle contrast image based on the     │
│  registered first speckle contrast       │
│  images                                  │
└─────────────────────────────────────────┘
```

FIG. 4B

**FIG. 5**

FIG. 6A    FIG. 6B    FIG. 6C

**FIG. 6D**

FIG. 7A

**FIG. 7B**

EP 4 213 705 B1

$$t = t_1$$

first wavelength

second wavelength

| 802 | illuminate target with coherent light of 1$^{st}$ wavelength |

↓

| 804 | detect 1$^{st}$ wavelength image |

↓

| 806 | calculate speckle contrast |

↓

| 808 | calculate corrected speckle contrast |

↓

| 810 | temporarily store (corrected) speckle contrast |

| 812 | illuminate target with coherent light of 2$^{nd}$ wavelength |

↓

| 814 | detect 2$^{nd}$ wavelength image |

↓

| 816 | calculate speckle contrast |

↓

| 818 | temporarily store 2$^{nd}$ wavelength image |

**FIG. 8**

EP 4 213 705 B1

FIG. 9

FIG. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2020045015 A1
- US 2019046232 A1
- EP 1324051 A1
- NL 2026240

### Non-patent literature cited in the description

- **W. HEEMAN et al.** Clinical applications of laser speckle contrast imaging: a review. *J. Biomed. Opt.,* 2019, vol. 24, 8
- **LERTSAKDADET et al.** Correcting for motion artefact in handheld laser speckle images. *Journal of biomedical optics,* March 2018, vol. 23 (2
- **P. MIAO et al.** High resolution cerebral blood flow imaging by registered laser speckle contrast analysis. *IEEE transactions on bio-medical engineering,* vol. 57 (5), 1152-1157